(19) Europäisches Patentamt
European Patent Office
Office européen des brevets

(11) EP 2 283 828 A1

(12) EUROPEAN PATENT APPLICATION

(43) Date of publication:
**16.02.2011 Bulletin 2011/07**

(51) Int Cl.:
***A61K 9/20*** *(2006.01)* ***A61K 31/517*** *(2006.01)*

(21) Application number: **10182729.3**

(22) Date of filing: **21.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **23.11.2005 GB 0523810**

(62) Document number(s) of the earlier application(s) in accordance with Art. 76 EPC:
**06808603.2 / 1 954 247**

(71) Applicant: **AstraZeneca AB**
**151 85 Södertälje (SE)**

(72) Inventors:
- **Cahill, Julie, Kay**
**Macclesfield, Cheshire SK10 2NA (GB)**
- **Cumberbatch, Daren, James**
**Macclesfield, Cheshire SK10 2NA (GB)**
- **Holt, David, John**
**Macclesfield, Cheshire SK10 2NA (GB)**
- **Richer, Sebastien**
**Macclesfield, Cheshire SK10 4TG (GB)**
- **Simpson, David, Bradley, Brook**
**Macclesfield, Cheshire SK10 2NA (GB)**
- **Swain, Elizabeth, Anne**
**Macclesfield, Cheshire SK10 2NA (GB)**

(74) Representative: **Burns, Tracy Anne et al**
**AstraZeneca AB**
**Global Intellectual Property**
**151 85 Södertälje (SE)**

Remarks:
This application was filed on 29-09-2010 as a divisional application to the application mentioned under INID code 62.

# (54) Pharmaceutical compositions

(57) Pharmaceutical compositions comprising AZD2171 or a pharmaceutically acceptable salt thereof, including pharmaceutical compositions comprising AZD2171 or a pharmaceutically acceptable salt and a plastic filler with a high surface area, excluding lactose.

EP 2 283 828 A1

## Description

The present invention relates to pharmaceutical compositions, particularly to pharmaceutical compositions containing AZD2171 or a pharmaceutically-acceptable salt thereof, to processes for the preparation of said pharmaceutical compositions, to said pharmaceutical compositions for use in the production of an antiangiogenic and/or vascular permeability reducing effect in a warm-blooded animal such as a human, to the use of said pharmaceutical compositions in the manufacture of a medicament for use in the production of an antiangiogenic and/or vascular permeability reducing effect in a warm-blooded animal such as a human and to a method for the production of an antiangiogenic and/or vascular permeability reducing effect in a warm-blooded animal such as a human which comprises the administration of such a pharmaceutical composition.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36:139-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1 (also referred to as VEGFR-1), the kinase insert domain-containing receptor, KDR (also referred to as VEGFR-2 or Flk-1), and another fms-like tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

VEGF is a key stimulus for vasculogenesis and angiogenesis. This cytokine induces a vascular sprouting phenotype by inducing endothelial cell proliferation, protease expression and migration, and subsequent organisation of cells to form a capillary tube (Keck, P.J., Hauser, S.D., Krivi, G., Sanzo, K., Warren, T., Feder, J., and Connolly, D.T., Science (Washington DC), 246: 1309-1312, 1989; Lamoreaux, W.J., Fitzgerald, M.E., Reiner, A., Hasty, K.A., and Charles, S.T., Microvasc. Res., 55: 29-42, 1998; Pepper, M.S., Montesano, R., Mandroita, S.J., Orci, L. and Vassalli, J.D., Enzyme Protein, 49: 138-162, 1996.). In addition, VEGF induces significant vascular permeability (Dvorak, H.F., Detmar, M., Claffey, K.P., Nagy, J.A., van de Water, L., and Senger, D.R., (Int. Arch. Allergy Immunol., 107: 233-235, 1995; Bates, D.O., Heald, R.I., Curry, F.E. and Williams, B. J. Physiol. (Lond.), 533: 263-272, 2001), promoting formation of a hyper-permeable, immature vascular network which is characteristic of pathological angiogenesis.

It has been shown that activation of KDR alone is sufficient to promote all of the major phenotypic responses to VEGF, including endothelial cell proliferation, migration, and survival, and the induction of vascular permeability (Meyer, M., Clauss, M., Lepple-Wienhues, A., Waltenberger, J., Augustin, H.G., Ziche, M., Lanz, C., Büttner, M., Rziha, H-J., and Dehio, C., EMBO J., 18: 363-374, 1999; Zeng, H., Sanyal, S. and Mukhopadhyay, D., J. Biol. Chem., 276: 32714-32719, 2001; Gille, H., Kowalski, J., Li, B., LeCouter, J., Moffat, B, Zioncheck, T.F., Pelletier, N. and Ferrara, N., J. Biol. Chem., 276: 3222-3230, 2001).

Quinazoline derivatives which are inhibitors of VEGF receptor tyrosine kinase are described in International Patent Application Publication No. WO 00/47212. AZD2171 is described in WO 00/47212 and is Example 240 therein. AZD2171 is 4-(4-fluoro-2-methyl-1H-indol-5-yloxy)-6-methoxy-7-(3-(pyrrolidin-1-yl)propoxy)quinazoline:

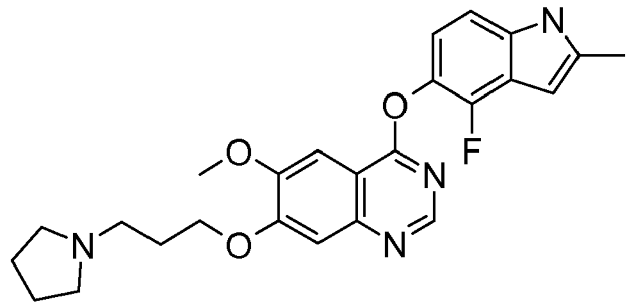

AZD2171

AZD2171 shows excellent activity in the *in vitro* (a) enzyme and (b) HUVEC assays that are described in WO 00/47212 (pages 80-83). The AZD2171 $IC_{50}$ values for inhibition of isolated KDR (VEGFR-2) and Flt-1 (VEGFR-1) tyrosine kinase activities in the enzyme assay were <1 nM and 5 ± 2 nM respectively. AZD2171 inhibits VEGF-stimulated endothelial cell proliferation potently ($IC_{50}$ value of 0.4 ± 0.2 nM in the HUVEC assay), but does not inhibit basal endothelial cell proliferation appreciably at a > 1250 fold greater concentration ($IC_{50}$ value is > 500 nM). The growth of a Calu-6 tumour xenograft in the *in vivo* solid tumour model described in WO 00/47212 (page 83) was inhibited by 49%$^{**}$, 69%$^{***}$ and 91%$^{***}$ following 28 days of once-daily oral treatment with 1.5, 3 and 6 mg/kg/day AZD2171 respectively ($P^{**}$<0.01, $P^{***}$<0.0001; one-tailed t test). AZD2171 has been shown to elicit broad-spectrum anti-tumour activity in a range of models following once-daily oral administration (Wedge et al., 2005, Cancer Research 65: 4389-4440).

A preferred salt of AZD2171 is AZD2171 maleate salt which is described in International Patent Application Publication No. WO 05/061488.

In WO 00/47212, Example 326 therein describes some pharmaceutical dosage forms of a compound of formula I.

In general, pharmaceutical compositions of the compounds of formula I in WO 00/47212 may be prepared in a conventional manner using conventional excipients. An oral dosage form (e.g. tablet, capsule, granules, pellets, lozenges etc.) of AZD2171 was sought for clinical trials and commercial use.

In developing an oral dosage form, particularly tablets, one must consider the mechanical properties of the active pharmaceutical ingredient and of any proposed excipients.

The mechanical properties of materials such as powders may be described in terms of the following characteristics:

(a) Hardness, or resistance to deformation, which can be measured by an indentation hardness test;
(b) Yield pressure (denoted P*y* and also known as yield stress), that is the point at which plastic deformation occurs, which can be measured by compaction studies;
(c) Strain Rate Sensitivity (SRS), that is the percent increase in yield pressure from slow to fast punch velocity, which can be measured by compaction studies; and
(d) Modulus of Elasticity, that is the ratio of stress to elongation (strain), which can be measured by numerous methods that are well known to a person skilled in the art of formulation.

Materials can, in general, be classified by the way in which they deform under compressive force, either by brittle fracture or by plastic deformation. The degree of deformation for a brittle material is independent of the rate and duration of the compression event (that is the compression applied), giving a strain rate sensitivity value for such materials of 0% (zero%). Deformation of a plastic material is dependent on the rate and duration of the compression event and this is described by the strain rate sensitivity.

When developing an oral dosage form, particularly a tablet formulation, it is usual to use a mixture of powders: some with brittle character to minimise the strain rate sensitivity and some with moderate plastic character to increase the surfaces available to form bonds during compression.

Tablet presses used in commercial manufacture typically run at much faster speeds than those used in research and development. As the speed of a press increases, the duration of the compression event (that is the period for which compression is applied; also known as dwell time) decreases. This has no impact on the compression of a brittle material with an SRS of say 0%, but for a plastic material, for which the deformation is dependent on the rate and duration of compression, a faster press would typically produce softer tablets. Therefore, an excess of plastic material in a formulation can lead to difficulties on scale up to the faster compression machines used in full scale production.

AZD2171 is a plastic material, for example on testing AZD2171 maleate was found to have a low yield pressure of 26.9 MPa (megapascals). Two further batches of AZD2171 maleate were tested and were found to have yield pressures of 29.2 MPa and 31.0 MPa. From the experiments done AZD2171 maleate demonstrates a yield pressure in the range 25-32 MPa.

The plasticity of the material makes the formulation of a solid oral dosage form of AZD2171 difficult. For example in tablet manufacture these properties can lead to problems in achieving suitably hard tablets, particularly when scaling up from the relatively slow tablet machines (with correspondingly long compression events) used in research and development for small numbers of tablets, to the faster compression machines (with short compression events) used in full scale commercial manufacture.

In order to counteract the plasticity of a drug such as AZD2171 a person skilled in the art would typically try to modify the material's properties with the addition of a complementary excipient such as a brittle filler. However this can limit the drug loading that can be achieved in a formulation, i.e. the percentage of active ingredient therein, and can lead to very large tablets which are difficult to swallow. Furthermore it was found that AZD2171 has relatively poor stability due to degradation by hydrolysis and oxidation. The brittle filler dibasic calcium phosphate dihydrate showed hydrolysis with AZD2171. It was therefore apparent that, due to the low stability of AZD2171, hydrated brittle fillers and hydrated excipients would be contraindicated for use in formulations of AZD2171. Initial test formulations of AZD2171 were therefore limited to the use of two plastic fillers namely lactose and mannitol. The test formulations comprised:

**Composition of AZD2171 5mg tablet cores (lactose formulation, = Mix E in Figure 1)**

| Ingredient | mg/tablet | Function |
|---|---|---|
| AZD2171 maleate | 6.30 | Active agent |
| Fast-Flo™ Lactose | 88.70 | Plastic Filler |
| Sodium starch glycolate | 4.00 | Disintegrant |
| Magnesium stearate | 1.00 | Lubricant |
| Total compression weight | 100 mg | |

**Composition of AZD2171 5mg tablet cores (mannitol formulation = Mix D in Figure 1)**

| Ingredient | Mg/tablet | Function |
|---|---|---|
| AZD2171 maleate | 6.30 | Active agent |
| Mannitol (Pearlitol™ 200 SD) | 88.70 | Plastic Filler |
| Sodium starch glycolate | 4.00 | Disintegrant |
| Magnesium stearate | 1.00 | Lubricant |
| Total compression weight | 100 mg | |

The conversion factor by weight from AZD2171 free base to AZD2171 maleate is 1.26, thus 6.3mg of AZD2171 maleate is equivalent to 5mg AZD2171 free base.

The lactose-based formulation (Mix E) produced a very soft tablet, but with no evidence of capping over a compression range consistent with the compaction pressures to be used in production, say 150-250 MPa (megapascals) (see Figure 1). The mannitol-based formulation (Mix D) also generated very soft tablets, which in addition showed a high capping tendency. The hardness of the mannitol-based formulation tablets peaked at about 200MPa (see Figure 1) and capping was observed at compaction pressures greater than 300MPa. The lactose-based formulation tablets were less compressible than the mannitol-based formulation tablets (see Figure 1). Although both the mannitol and lactose-based formulations produced tablets with an acceptable appearance, they were not sufficiently robust to withstand subsequent processing and handling.

These problems were found using relatively slow compression machines in development; they would be exacerbated on scale up to faster compression machines.

The object of the present invention is to provide pharmaceutical compositions of AZD2171 that have the required good compression properties, requisite hardness, resistance to friability, and that have the required good disintegration and dissolution properties, that allow high drug loading, and that do not cap when made into tablets.

Preferably the pharmaceutical compositions of AZD2171 are solid oral dosage forms of AZD2171, particularly tablets of AZD2171.

More preferably the pharmaceutical compositions of AZD2171 are solid oral dosage forms of AZD2171 maleate,

particularly tablets of AZD2171 maleate.

Surprisingly, we have found that a formulation of AZD2171 with a plastic filler with a high surface area, excluding lactose, as the principal excipient has particularly advantageous properties. This is contrary to expectation because AZD2171 is itself a plastic material so one would expect that adding further plastic material to a formulation of AZD2171 would be detrimental to the compression and disintegration properties at a suitable tablet hardness. It is known in the art that for any plastic material there is a balance between hardness and disintegration properties. The greater the compaction pressure applied, the harder a tablet will be, the lower its surface area will be, because its porosity will be reduced, but the longer the disintegration time it will have. Thus the greater the compaction pressure applied, the harder a tablet will be and the less porous it will be but the longer the disintegration time it will have. Conversely the less the compaction pressure applied, the lower the hardness of the tablet but the shorter the disintegraton time.

According to the present invention there is provided a pharmaceutical composition comprising AZD2171 or a pharmaceutically acceptable salt thereof and a plastic filler with a high surface area, excluding lactose.

Plastic fillers with a high surface area include Parteck M™ mannitol and silicified microcrystalline cellulose.

In one embodiment of the present invention the plastic filler with a high surface area excluding lactose is Parteck M™ mannitol.

In one embodiment of the present invention the plastic filler with a high surface area excluding lactose is silicified microcrystalline cellulose.

Unexpectedly and surprisingly, we have found that use of a plastic filler with a high surface area excluding lactose, for example silicified microcrystalline cellulose (SMCC) in an AZD2171 formulation results in tablets with improved compression properties in terms of hardness and resistance to capping, whilst still maintaining good dissolution and disintegration properties.

A preferred form of silicified microcrystalline cellulose is Prosolv® (J. Rettenmaier & Sohne GmbH + Co.KG (JRS PHARMA), Rosenberg, Germany). Prosolv SMCC® comprises 98% microcrystalline cellulose and 2% colloidal silicon dioxide and is described at the JRS Pharma website: http://www.jrspharma.com/excip_prosolv.php. Different grades of Prosolv® are available, for example Prosolv® HD 90, Prosolv SMCC® 50, Prosolv SMCC® 90 and Prosolv SMCC® 90LM.

Prosolv® HD 90 is a silicified high density microcrystalline cellulose with a median particle size in the region of 90μm.

Prosolv SMCC® 50 is a silicified microcrystalline cellulose with a median particle size in the region of 50μm.

Prosolv SMCC® 90 is a silicified microcrystalline cellulose with a median particle size in the region of 90μm.

Prosolv SMCC® 90LM is a low moisture silicified microcrystalline cellulose with a median particle size in the region of 90μm.

For example the total surface area of Prosolv SMCC® 50 was measured in one experiment and found to be 6.00$m^2$/g. It will be recognised by a person skilled in the art that the measurement of total surface area can vary slightly from experiment to experiment and between different samples of the same material. The figure of 6.00$m^2$/g is given as one representative example of mean total surface area of Prosolv SMCC® 50.

According to the present invention there is provided a pharmaceutical composition comprising AZD2171 or a pharmaceutically acceptable salt thereof and a plastic filler that is silicified microcrystalline cellulose.

In one embodiment of the present invention the form of silicified microcrystalline cellulose is Prosolv®.

In one embodiment of the present invention the form of silicified microcrystalline cellulose is Prosolv® HD 90.

In one embodiment of the present invention the form of silicified microcrystalline cellulose is Prosolv SMCC® 50.

In one embodiment of the present invention the form of silicified microcrystalline cellulose is Prosolv SMCC® 90.

In one embodiment of the present invention the form of silicified microcrystalline cellulose is Prosolv SMCC® 90LM.

A formulation of AZD2171 maleate with Prosolv® is shown below and is exemplified in Example 5 hereinafter.

**Composition of AZD2171 30mg tablet cores (9.0 mm normal concave (N/C) round)**

| Ingredient | mg/tab | Function |
|---|---|---|
| AZD2171 maleate | 37.8 | Active agent |
| Prosolv SMCC® 90 | 245.7 | Plastic filler |
| Sodium starch glycolate | 12.0 | Disintegrant |
| Magnesium stearate | 4.5 | Lubricant |
| **Total** | **300 mg** | |

One way to increase surface area is to increase porosity, porous materials have a high surface area.

Surprisingly, we have found that a formulation of AZD2171 with a plastic filler with an open porous structure excluding lactose as the principal excipient has particularly advantageous properties. Unexpectedly and surprisingly, we have found that use of a plastic filler with an open porous structure excluding lactose, for example Parteck M™ mannitol, in an AZD2171 formulation results in tablets with improved compression properties in terms of hardness and resistance to capping, whilst still maintaining good dissolution and disintegration properties.

According to the present invention there is provided a pharmaceutical composition comprising AZD2171 or a pharmaceutically acceptable salt thereof and a plastic filler with an open porous structure excluding lactose.

A plastic diluent or filler with an open porous structure excluding lactose is preferably Parteck M™ mannitol. Merck KGaA, Darmstadt, Germany and Merck Chemicals Ltd. UK, describe Parteck M™ mannitol http://www.merck.de/servlet/PB/menu/1228300/index.html as having a 'needle-like microstructure' and recommend it for increasing the hardness of formulations. Compared to granular mannitol or conventional spray-dried mannitol they say that Parteck M™ mannitol produces increased hardness in equivalent formulations. However they do not say that it is recommended for use with plastic materials.

Parteck M™ mannitol is available in different grades based on particle size. For example Parteck M™ 200 mannitol and Parteck M™ 300 mannitol have mean particle sizes of 200μm and 300μm respectively.

In one embodiment of the present invention Parteck M™ 200 mannitol is used.
In one embodiment of the present invention Parteck M™ 300 mannitol is used.
In one embodiment of the present invention Parteck M™ 100 mannitol is used.

**Composition of AZD2171 5mg tablet cores (Parteck M™ mannitol formulation, = Mix C in Figure 1)**

| Ingredient | mg/tablet | Function |
|---|---|---|
| AZD2171 maleate | 6.30 | Active agent |
| Parteck M™ mannitol | 88.70 | Plastic filler |
| Sodium starch glycolate | 4.00 | Disintegrant |
| Magnesium stearate | 1.00 | Lubricant |
| Total compression weight | 100 mg | |

An equivalent formulation of AZD2171 maleate with Parteck M™ mannitol is exemplified in Example 1 hereinafter.

The compression profiles of the test formulations of AZD2171 maleate with Fast-Flo™ lactose, Pearlitol™ mannitol and Parteck M™ mannitol are shown in Figure 1. The formulation with Parteck M™ mannitol (Mix C) has more capacity for increased compaction pressure and is clearly better than both the lactose formulation (Mix E) and the alternative grade mannitol formulation (Mix D).

The compression profiles of test formulations of AZD2171 maleate with Pearlitol™200 mannitol, Parteck M™ mannitol and Prosolv SMCC® 50 are shown in Figure 3. Pearlitol™200 mannitol has a surface area less than 1.5$m^2$/g and is therefore not classed as a plastic filler with a high surface area. The formulations with Prosolv SMCC® 50 and Parteck M™ mannitol have more capacity for increased compaction pressure and are clearly better than the Pearlitol™200 mannitol formulation.

For example in one experiment the total surface area of Pearlitol™200 mannitol was measured and found to be 0.38$m^2$/g.

For example in one experiment the total surface area of Parteck M™ 200 mannitol was measured and found to be 3.52$m^2$/g.

It will be recognised by a person skilled in the art that the measurement of total surface area can vary slightly from experiment to experiment and between different samples of the same material. The figures above are two representative examples of total surface area for Pearlitol™200 mannitol and Parteck M™ 200 mannitol.

Although the formulation with Prosolv SMCC® described above, and exemplified in Example 5, has advantageous compression and disintegration properties it was found that at compaction pressures greater than about 150 MPa capping occurred.

Although the formulation with Parteck M™ mannitol described above has advantageous compression and disintegration properties it was found that if the AZD2171 loading was increased above about 5%, ie if the amount of AZD2171 was increased to above about 5%, then the capping tendency of the formulation also increased. In order to try to counteract the plastic nature of the formulation and reduce the capping tendency, the addition of a brittle filler was used. Since AZD2171 is susceptible to hydrolysis an anhydrous brittle filler was sought. The following brittle fillers were

evaluated:

dibasic calcium phosphate, anhydrous, unmilled and milled grades;
tribasic calcium phosphate, anhydrous; and
calcium carbonate, anhydrous.

Unexpectedly and surprisingly we have found that a pharmaceutical composition comprising AZD2171 or a pharmaceutically acceptable salt thereof, a plastic filler with a high surface area, excluding lactose, and a brittle filler with a low surface acidity gives better stability and gives improved compression properties in terms of hardness and resistance to capping, whilst still maintaining good dissolution and disintegration properties.

According to another aspect of the present invention there is provided a pharmaceutical composition comprising AZD2171 or a pharmaceutically acceptable salt thereof, a plastic filler with a high surface area excluding lactose and a brittle filler with a low surface acidity.

In one aspect of the present invention the plastic filler with a high surface area is SMCC, preferably Prosolv®, and the brittle filler with a low surface acidity is dibasic calcium phosphate anhydrous milled grade.

A formulation of AZD2171 maleate with Prosolv SMCC® and dibasic calcium phosphate anhydrous milled grade is shown below and is exemplified in Example 6 hereinafter.

**Composition of AZD2171 30mg tablet cores (9.0 mm N/C round Prosolv®/dibasic calcium phosphate formulation)**

| Ingredient | mg/tab | Function |
|---|---|---|
| AZD2171 maleate | 37.8 | Active agent |
| Prosolv SMCC® 50 | 200.7 | Plastic filler |
| Dibasic calcium phosphate anydrous milled grade | 45.0 | Brittle filler |
| Sodium starch glycolate | 12.0 | Disintegrant |
| Magnesium stearate | 4.5 | Lubricant |
| **Total** | **300 mg** | |

Unexpectedly and surprisingly we have found that a pharmaceutical composition comprising AZD2171 or a pharmaceutically acceptable salt thereof, a plastic filler with an open porous structure excluding lactose and a brittle filler with a low surface acidity gives better stability and gives improved compression properties in terms of hardness and resistance to capping, whilst still maintaining good dissolution and disintegration properties.

According to another aspect of the present invention there is provided a pharmaceutical composition comprising AZD2171 or a pharmaceutically acceptable salt thereof, a plastic filler with an open porous structure excluding lactose and a brittle filler with a low surface acidity.

In one aspect of the present invention the brittle filler with a low surface acidity is dibasic calcium phosphate anhydrous milled grade.

In one aspect of the present invention the brittle filler with a low surface acidity is dibasic calcium phosphate anhydrous milled grade and the plastic filler with an open porous structure is Parteck M™ mannitol.

**Composition of AZD2171 30mg tablet cores (Parteck M™/dibasic calcium phosphate formulation)**

| Ingredient | mg/tab | Function |
|---|---|---|
| AZD2171 maleate | 37.80 | Active agent |
| Parteck M™ mannitol | 200.70 | Plastic filler |
| Dibasic calcium phosphate, anhydrous, milled grade (Calipharm A™) | 45.00 | Brittle filler |
| Sodium starch glycolate | 12.00 | Disintegrant |
| Magnesium stearate | 4.50 | Lubricant |
| Total | 300 mg | |

This formulation of AZD2171 maleate with Parteck M™ mannitol and Calipharm A™ is exemplified in Example 2 hereinafter.

In Figure 2 the relative tablet hardness at increasing compaction pressures is shown for formulations of AZD2171 maleate substantially the same as that of Example 2 but with different amounts of dibasic calcium phosphate anhydrous milled grade: AZD2171 maleate with 5% dibasic calcium phosphate anhydrous milled grade, and AZD2171 maleate with 20% dibasic calcium phosphate anhydrous milled grade. The last point on each curve is when capping was seen. For AZD2171 maleate alone, capping was seen at a relatively low compaction pressure but for both the formulations with dibasic calcium phosphate anhydrous milled grade, capping only occurred at a much higher compaction pressure and one beyond that required for manufacturing processes.

According to another aspect of the present invention there is provided a pharmaceutical composition comprising AZD2171 or a pharmaceutically acceptable salt thereof and a brittle filler with a low surface acidity.

According to another aspect of the present invention there is provided a pharmaceutical composition comprising AZD2171 maleate and dibasic calcium phosphate anhydrous milled grade.

In Figure 4 the relative tablet hardness is shown for three formulations of AZD2171 maleate containing: 15% of dibasic calcium phosphate and Pearlitol™ 300DC mannitol (a plastic filler with a surface area of less than $1.5m^2/g$), 15% of dibasic calcium phosphate and Parteck M™ mannitol and Prosolv SMCC® 90 (Example 5). The last point on the curves for the Prosolv® and Pearlitol™ 300DC formulations is when capping was seen. Capping was not seen with the Parteck M™ mannitol formulation. For the Pearlitol™ 300DC mannitol formulation, capping was seen at a relatively low compaction pressure but for the Parteck M™ mannitol formulation capping was not seen at any of the compaction pressures tested including those higher than would be required for manufacturing processes. Although the Prosolv SMCC® 90 tablets demonstrated the greatest hardness at lower compaction pressures, the formulation with Prosolv SMCC® 90 contained no dibasic calcium phosphate and capping occurred at about 150 MPa.

Although the formulation described above and in Example 2 has good compression properties and allows drug loading greater than about 5% it was found that the tablets had poor weight uniformity due to poor flow properties of the formulation. As a consequence of the poor weight uniformity the tablets also had appearance problems and were easily damaged and friable.

Unexpectedly and surprisingly it was found that addition of more plastic material in the form of a secondary plastic filler improved the appearance of the tablets whilst maintaining good compression properties in terms of hardness and resistance to capping, and good dissolution and disintegration properties.

According to another aspect of the present invention there is provided a pharmaceutical composition comprising AZD2171 or a pharmaceutically acceptable salt thereof, a plastic filler with a high surface area excluding lactose, a brittle filler with a low surface acidity and optionally a secondary plastic filler.

According to another aspect of the present invention there is provided a pharmaceutical composition comprising AZD2171 or a pharmaceutically acceptable salt thereof, a plastic filler with an open porous structure excluding lactose, a brittle filler with a low surface acidity and optionally a secondary plastic filler.

According to another aspect of the present invention there is provided a pharmaceutical composition comprising AZD2171 or a pharmaceutically acceptable salt thereof, a plastic filler with an open porous structure excluding lactose, a brittle filler with a low surface acidity and a secondary plastic filler.

Secondary plastic fillers include starch and microcrystalline cellulose.

In one aspect of the present invention the secondary plastic filler is microcrystalline cellulose.

**Composition of AZD2171 30mg tablet cores (microcrystalline cellulose formulation)**

| Ingredient | mg/tab | Function |
|---|---|---|
| AZD2171 maleate | 37.80 | Active agent |
| Parteck M™ mannitol | 163.20 | Plastic filler |
| Dibasic calcium phosphate, anhydrous milled grade (Calipharm A™) | 45.00 | Brittle filler |
| Microcrystalline cellulose (Avicel™) | 37.50 | Secondary plastic filler |
| Sodium starch glycolate | 12.00 | Disintegrant |
| Magnesium stearate | 4.50 | Lubricant |
| Total | 300 mg | |

This formulation of AZD2171 maleate with Parteck M™ mannitol, Calipharm A™ and Avicel™ is exemplified in Example 3 hereinafter.

Surprisingly the use of a secondary plastic filler such as microcrystalline cellulose not only gives tablets with a better appearance but also reduces the capping tendency of the formulation without adversely prolonging disintegration times. Thus for Example 2 the tablets had a disintegration time of 1 minute whereas for Example 3 the tablets had a

disintegration time of 2.5 minutes, still well within the acceptable range of less than 15 minutes.

Prosolv® is a high functionality excipient (HFE). HFEs are multifunctional, i.e. they combine more than one function in a single ingredient. Prosolv® is capable of providing the functions of both the primary and secondary plastic fillers, that is to say the plastic filler with a high surface area and the secondary plastic filler, in pharmaceutical compositions containing AZD2171 or a pharmaceutically acceptable salt thereof.

According to one aspect of the present invention in formulations containing Prosolv® no secondary plastic filler is added.

Suitably, the weight ratio of AZD2171 or a pharmaceutically acceptable salt thereof, especially AZD2171 maleate to the plastic filler with a high surface area excluding lactose is from 4:1 to 1:950, for example from 1:1 to 1:500, preferably from 1:2.5 to 1:250, particularly from 1:2.5 to 1:150, more particularly from 1:2.5 to 1:10.

Suitably, the weight ratio of the brittle filler with a low surface acidity to the plastic filler with a high surface area excluding lactose is from 2:1 1 to 1:950, for example from 1:1 to 1:50, more particularly from 1:2 to 1:15, especially from 1:2 to 1:7.

Suitably the pharmaceutical composition contains for example, from 15 to 95%, particularly from 30 to 85%, more particularly from 35 to 75%, especially from 45 to 70% by weight, based upon the total weight of the composition, of a plastic filler with a high surface area excluding lactose.

Suitably, the weight ratio of AZD2171 or a pharmaceutically acceptable salt thereof, especially AZD2171 maleate to the plastic filler with an open porous structure excluding lactose is from 4:1 1 to 1:950, for example from 1:1 to 1:500, more particularly from 1:2.5 to 1:250, still more particularly from 1:2.5 to 1:150, still more particularly from 1:2.5 to 1:10.

Suitably, the weight ratio of the brittle filler with a low surface acidity to the plastic filler with an open porous structure excluding lactose is from 2:1 to 1:950, for example from 1:1 to 1:50, more particularly from 1:2 to 1:15.

Where a secondary plastic filler is used suitably, the weight ratio of AZD2171 or a pharmaceutically acceptable salt thereof, especially AZD2171 maleate to the secondary plastic filler is from 4:1 to 1:950, preferably from 4:1 to 1:50, for example from 2:1 to 1:50, more particularly from 2:1 to 1:15, especially from 2:1 to 1:5.

Suitably the pharmaceutical composition contains for example, from 15 to 95%, particularly from 30 to 80%, more particularly from 35 to 75%, especially from 45 to 70%, more especially from 45 to 65% by weight, based upon the total weight of the composition, of a plastic filler with an open porous structure excluding lactose.

Suitably the pharmaceutical composition contains for example, from 1 to 50%, particularly from 2 to 40%, more particularly from 5 to 30%, especially from 10 to 20% by weight, based upon the total weight of the composition, of a brittle filler with a low surface acidity.

Where a secondary plastic filler is used suitably the pharmaceutical composition contains for example, from 1 to 50%, particularly from 2 to 40%, more particularly from 5 to 30%, especially from 10 to 20% by weight, based upon the total weight of the composition, of a secondary plastic filler.

The pharmaceutical compositions of the present invention are advantageously presented in unit dosage form. AZD2171 will normally be administered to a warm-blooded animal at a unit dose within the range 1-50mg per square metre body area of the animal, for example approximately 0.03-1.5 mg/kg in a human. A unit dose in the range, for example, 0.01-1.5mg/kg, preferably 0.03-0.5mg/kg is envisaged and this is normally a therapeutically-effective dose. A unit dosage form such as a tablet or capsule will usually contain, for example 1-60mg of active ingredient. Preferably a daily dose in the range of 0.03-0.5mg/kg is employed.

Thus the composition may contain from 0.5mg to 100mg of AZD2171 or a pharmaceutically acceptable salt thereof. Suitable quantities of AZD2171 or a pharmaceutically acceptable salt thereof include, for example, 0.5, 1, 5, 10, 15, 20, 25, 30, 40, 45, 50, 60 or 100mg, depending upon the dose required and the particular form of the pharmaceutical composition. In one aspect of the present invention the pharmaceutical composition contains 10, 15, 20, 30, 45, 60 or 90mg of AZD2171 or a pharmaceutically acceptable salt thereof. In one aspect of the present invention the pharmaceutical composition contains the equivalent of 10, 15, 20, 30, 45, 60 or 90mg of AZD2171.

Typically AZD2171 or a pharmaceutically acceptable salt thereof especially AZD2171 maleate will be present in an amount within the range of from 0.5 to 99%, and suitably from 0.5 to 50%, for example from 0.5 to 30%, preferably from 0.5 to 20% and especially from 0.5 to 15% by weight of the pharmaceutical composition.

According to another aspect of the present invention there is provided a pharmaceutical composition comprising AZD2171 or a pharmaceutically acceptable salt thereof, a plastic filler with a high surface area excluding lactose, a brittle filler with a low surface acidity, optionally a secondary plastic filler, and a disintegrant.

According to another aspect of the present invention there is provided a pharmaceutical composition comprising AZD2171 or a pharmaceutically acceptable salt thereof, a plastic filler with an open porous structure excluding lactose, a brittle filler with a low surface acidity, optionally a secondary plastic filler and a disintegrant.

According to another aspect of the present invention there is provided a pharmaceutical composition comprising AZD2171 or a pharmaceutically acceptable salt thereof, a plastic filler with an open porous structure excluding lactose, a brittle filler with a low surface acidity, a secondary plastic filler and a disintegrant.

Suitable disintegrants include those known in the art of formulation, such as those listed in The Handbook of Pharmaceutical Excipients, 4th edition, eds Rowe, R. C. et al, Pharmaceutical Press, 2003. Preferred disintegrants include sodium starch glycolate, croscarmellose sodium and starch.

A suitable weight ratio of AZD2171 or a pharmaceutically acceptable salt thereof especially AZD2171 maleate to disintegrant is from 25:1 to 0.0125:1, particularly from 10:1 to 0.1:1, more particularly from 8:1 to 0.5:1 and still more particularly from 3.5:1 to 1.25:1.

Suitably the pharmaceutical composition will contain from 0.01 to 10%, for example from 1 to 8%, particularly from 2 to 7% and more particularly from 3 to 6% by weight of disintegrant.

Other additional excipients may optionally be included in a pharmaceutical composition according to the present invention. Additional excipients include for example lubricants.

According to another aspect of the present invention there is provided a pharmaceutical composition comprising AZD2171 or a pharmaceutically acceptable salt thereof, a plastic filler with a high surface area excluding lactose, a brittle filler with a low surface acidity, optionally a secondary plastic filler, a disintegrant and a lubricant.

According to another aspect of the present invention there is provided a pharmaceutical composition comprising AZD2171 or a pharmaceutically acceptable salt thereof, a plastic filler with an open porous structure excluding lactose, a brittle filler with a low surface acidity, optionally a secondary plastic filler, a disintegrant and a lubricant.

According to another aspect of the present invention there is provided a pharmaceutical composition comprising AZD2171 or a pharmaceutically acceptable salt thereof, a plastic filler with an open porous structure excluding lactose, a brittle filler with a low surface acidity, a secondary plastic filler, a disintegrant and a lubricant.

In one aspect of the present invention a lubricant is magnesium stearate.

Suitably one or more lubricants will be present in an amount of from 0.1 to 10% by weight, for example from 0.5 to 2.0% by weight.

According to another aspect of the present invention there is provided a pharmaceutical composition comprising AZD2171 or a pharmaceutically acceptable salt thereof, a plastic filler with a high surface area excluding lactose, a brittle filler with a low surface acidity, optionally a secondary plastic filler, a disintegrant, a lubricant and a binder.

According to another aspect of the present invention there is provided a pharmaceutical composition comprising AZD2171 or a pharmaceutically acceptable salt thereof, a plastic filler with an open porous structure excluding lactose, a brittle filler with a low surface acidity, optionally a secondary plastic filler, a disintegrant, a lubricant and a binder.

According to another aspect of the present invention there is provided a pharmaceutical composition comprising AZD2171 or a pharmaceutically acceptable salt thereof, a plastic filler with an open porous structure excluding lactose, a brittle filler with a low surface acidity, a secondary plastic filler, a disintegrant, a lubricant and a binder.

In one aspect of the present invention a binder is polyvinyl pyrollidone (povidone, PVP) or hydroxypropyl methylcellulose (HPMC).

Suitably one or more binders will be present in an amount of from 0.5 to 50% by weight, for example from 1 to 10% by weight.

Further additional excipients which may be added include preservatives, stabilisers, anti-oxidants, silica flow conditioners, antiadherents or glidants.

According to another aspect of the present invention there is provided a solid pharmaceutical composition for oral administration comprising AZD2171 or a pharmaceutically acceptable salt thereof according to any of the embodiments described herein.

A particular pharmaceutical composition of the present invention comprises:

(a) from 0.1 to 50 (particularly from 0.5 to 30) parts AZD2171 or a pharmaceutically acceptable salt thereof especially AZD2171 maleate;
(b) from 15 to 95 (particularly from 35 to 85) parts of a plastic filler with a high surface area excluding lactose; and
(c) from 0 to 50 (particularly from 10 to 20) parts of a brittle filler with a low surface acidity;

wherein all parts are by weight and the sum of the parts (a)+(b)+(c)=100.

Another particular pharmaceutical composition of the present invention comprises:

(a) from 0.1 to 50 (particularly from 0.5 to 30) parts AZD2171 or a pharmaceutically acceptable salt thereof especially AZD2171 maleate;
(b) from 15 to 95 (particularly from 35 to 85) parts of a plastic filler with a high surface area excluding lactose;
(c) from 0 to 50 (particularly from 10 to 20) parts of a brittle filler with a low surface acidity;
(d) from 0 to 50 (particularly from 10 to 20) parts of a secondary plastic filler;
(e) from 0.1 to 10 (particularly from 1 to 10) parts of a disintegrant; and
(f) from 0.01 to 8 (particularly from 0.05 to 5) parts of a lubricant;

wherein all parts are by weight and the sum of the parts (a)+(b)+(c)+(d)+(e)+(f) = 100. Another particular pharmaceutical composition of the present invention comprises:

(a) from 0.1 to 50 (particularly from 0.5 to 30) parts AZD2171 or a pharmaceutically acceptable salt thereof especially AZD2171 maleate;
(b) from 15 to 95 (particularly from 45 to 75) parts of a plastic filler with an open porous structure excluding lactose; and
(c) from 1 to 50 (particularly from 10 to 20) parts of a brittle filler with a low surface acidity;

wherein all parts are by weight and the sum of the parts (a)+(b)+(c)=100.

Another particular pharmaceutical composition of the present invention comprises:

(a) from 0.1 to 50 (particularly from 0.5 to 30) parts AZD2171 or a pharmaceutically acceptable salt thereof especially AZD2171 maleate;
(b) from 15 to 95 (particularly from 45 to 65) parts of a plastic filler with an open porous structure excluding lactose;
(c) from 1 to 50 (particularly from 10 to 20) parts of a brittle filler with a low surface acidity;
(d) from 0 to 50 (particularly from 10 to 20) parts of a secondary plastic filler;
(e) from 0.1 to 10 (particularly from 1 to 10) parts of a disintegrant; and
(f) from 0.01 to 8 (particularly from 0.05 to 5) parts of a lubricant;

wherein all parts are by weight and the sum of the parts (a)+(b)+(c)+(d)+(e)+(f) = 100. Another particular pharmaceutical composition of the present invention comprises:

(a) from 0.1 to 50 (particularly from 0.5 to 30) parts AZD2171 or a pharmaceutically acceptable salt thereof especially AZD2171 maleate;
(b) from 15 to 95 (particularly from 45 to 65) parts of a plastic filler with an open porous structure excluding lactose;
(c) from 1 to 50 (particularly from 10 to 20) parts of a brittle filler with a low surface acidity;
(d) from 1 to 50 (particularly from 10 to 20) parts of a secondary plastic filler;
(e) from 0.1 to 10 (particularly from 1 to 10) parts of a disintegrant; and
(f) from 0.01 to 8 (particularly from 0.05 to 5) parts of a lubricant;

wherein all parts are by weight and the sum of the parts (a)+(b)+(c)+(d)+(e)+(f) = 100.

When the pharmaceutical composition according to the invention is a solid dosage form such as a tablet, pellet or granules the solid composition optionally further comprises a suitable coating, for example a film coating. A coating can be used to provide protection against, for example, moisture ingress or degradation by light, to colour the formulation, or to modify or control the release of AZD2171 from the formulation.

According to one aspect of the present invention there is provided a pharmaceutical composition comprising a core comprising AZD2171 or a pharmaceutically acceptable salt thereof, a plastic filler with a high surface area excluding lactose, a brittle filler with a low surface acidity, and optionally a secondary plastic filler, and a coating.

According to one aspect of the present invention there is provided a pharmaceutical composition comprising a core comprising AZD2171 or a pharmaceutically acceptable salt thereof, a plastic filler with an open porous structure excluding lactose, a brittle filler with a low surface acidity, and optionally a secondary plastic filler, and a coating.

According to one aspect of the present invention there is provided a pharmaceutical composition comprising a core comprising AZD2171 or a pharmaceutically acceptable salt thereof, a plastic filler with an open porous structure excluding lactose, a brittle filler with a low surface acidity, and a secondary plastic filler, and a coating.

According to one aspect of the present invention there is provided a pharmaceutical composition comprising a core comprising AZD2171 or a pharmaceutically acceptable salt thereof, a plastic filler with a high surface area excluding lactose, a brittle filler with a low surface acidity, optionally a secondary plastic filler, a disintegrant and a lubricant, and a coating.

According to one aspect of the present invention there is provided a pharmaceutical composition comprising a core comprising AZD2171 or a pharmaceutically acceptable salt thereof, a plastic filler with an open porous structure excluding lactose, a brittle filler with a low surface acidity, optionally a secondary plastic filler, a disintegrant and a lubricant, and a coating.

According to one aspect of the present invention there is provided a pharmaceutical composition comprising a core comprising AZD2171 or a pharmaceutically acceptable salt thereof, a plastic filler with an open porous structure excluding lactose, a brittle filler with a low surface acidity, a secondary plastic filler, a disintegrant and a lubricant, and a coating.

The term 'good compression properties' relates to the mechanical properties of a material or mixture of powdered components. A material with good compression properties will consolidate under compressive force to form a "compact"

which is of the requisite hardness, is not prone to damage during mechanical agitation, is not prone to capping, and can be formed at higher processing speeds where strain rates are higher and time available for compression is shorter.

The term 'requisite hardness' means sufficient mechanical strength, which will prevent a compact from becoming damaged during subsequent processing or transport. This is related to the size of the tablet and when measured in kiloponds (kp) is typically at least 0.8 x the diameter of the tablet (mm), preferably at least 1 x tablet diameter, more preferably at least 1.1 x tablet diameter, particularly at least 1.2 x tablet diameter, especially at least 1.3 x tablet diameter. The greater the hardness the more robust the tablet is but at very high levels of hardness disintegration times can be unduly long.

The term 'suitable tablet hardness' means a tablet that is hard enough to withstand processing whilst having an appropriate disintegration time.

The term 'capping' means the complete or partial separation of a saucer-shaped disc from the top or bottom surface of a tablet during compression of the material to form a tablet or during subsequent processes and/or handling. Capping is described in Carstensen, J. T., Solid pharmaceutics: mechanical properties and rate phenomena., Academic press, New York (1980) and in Sheth et al., Pharmaceutical dosage forms: Tablets. Vol 1. Ed Liebermann and Lachmann, Pub. Marcel Dekker, New York (1980). If a material has a high capping tendency then it will cap at lower compaction pressures.

The term 'friability' means the phenomenon whereby tablet surfaces are damaged and/or show evidence of cracking or breakage when subjected to mechanical agitation (e.g. during processing, handling or transportation).

The term "disintegration" means the process whereby a tablet breaks down into its constituent particles when in contact with a fluid.

The term "appropriate disintegration time" means for example a disintegration time of less than 15 mintues, conveniently less than 12 minutes, advantageously less than 10 minutes, suitably less than 9 minutes, preferably less than 8 minutes, more preferably less than 7 minutes, particularly less than 6 minutes, more particularly less than 5 minutes and especially less than 4 minutes.

The term "dissolution" means the process by which drug particles dissolve. In order for a drug to be absorbed it must first be dissolved in the fluid at the site of absorption.

The term "drug loading" or "AZD2171 loading" means the amount of AZD2171 or a pharmaceutically acceptable salt thereof in the solid oral dosage form eg a tablet. For example, a drug loading of 10% AZD2171 produces a 30 mg tablet with a compression weight, i.e. total weight of the tablet, of 300 mg which is 9.0mm in diameter.

The term 'brittle' as in 'brittle filler or brittle diluent' means that the material has a yield pressure (P*y*) of greater than 300MPa and/or a strain rate sensitivity of from 0 to 10%.

The term 'plastic' as in 'plastic filler or plastic diluent' means that the material has a yield pressure of less than 150MPa, preferably less than 120MPa, especially less than 100MPa and/or has a strain rate sensitiviy of from 10 to 150%, preferably from 10 to 100%, especially from 30 to 80%.

A 'plastic filler with a high surface area' may be a plastic filler with an open porous structure or may be a plastic filler with medium or low porosity but nevertheless a high surface area.

The term 'high surface area' means a total surface area of greater than about 1.5$m^2$/g, preferably greater than about 1.8$m^2$/g, more preferably greater than about 1.9$m^2$/g, particularly greater than about 2.0$m^2$/g, more particularly greater than about 2.5$m^2$/g, especially greater than about 2.8$m^2$/g.

In one embodiment of the present invention 'high surface area' means a total surface area in the range of about 1.5$m^2$/g to about 10$m^2$/g.

In one embodiment of the present invention 'high surface area' means a total surface area in the range of 1.5$m^2$/g to 10$m^2$/g.

In one embodiment of the present invention 'high surface area' means a total surface area in the range of about 2.0$m^2$/g to about 10$m^2$/g.

In one embodiment of the present invention 'high surface area' means a total surface area in the range of 2.0$m^2$/g to 10$m^2$/g.

In one embodiment of the present invention 'high surface area' means a total surface area in the range of about 2.5$m^2$/g to about 10$m^2$/g.

In one embodiment of the present invention 'high surface area' means a total surface area in the range of 2.5$m^2$/g to 10$m^2$/g.

A plastic filler with an 'open porous structure excluding lactose' means a plastic filler that is not lactose and that has a high porosity. One way of increasing the porosity of a plastic filler is to have an internal structure that maximises the internal surface area of the filler, for example by having a needle-like microstructure as in Parteck M™ mannitol.

The term 'low surface acidity' means a surface pH that is greater than about pH 5.0, especially a surface acidity greater than about pH 5.5.

The present invention relates to pharmaceutical compositions comprising AZD2171 or a pharmaceutically acceptable salt thereof.

Salts of AZD2171 for use in pharmaceutical compositions will be pharmaceutically acceptable salts, but other salts may be useful in the production of AZD2171 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

A particularly preferred salt is AZD2171 maleate salt.

AZD2171 may be synthesised according to any of the known processes for making AZD2171. For example AZD2171 may be made according to any of the processes described in WO 00/47212; for example those described in Example 240 therein. AZD2171 maleate salt may be synthesised according to any of the known processes for making AZD2171 maleate salt. For example AZD2171 maleate salt may be made according to any of the processes described in WO 05/061488.

In one aspect of the present invention AZD2171 maleate salt is used for preparing the pharmaceutical compositions of the present invention.

In another aspect of the present invention AZD2171 anhydrous free base is used for preparing the pharmaceutical compositions of the present invention.

The pharmaceutical compositions of the present invention may be prepared by conventional wet or dry granulation, or dry blending, compression and then optionally, if a coating is desired, with film coating processes. Preferably the pharmaceutical compositions of the present invention are prepared by dry granulation.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with a high surface area excluding lactose, a brittle filler with a low surface acidity, optionally a secondary plastic filler and optionally other excipients, to produce a homogenous mix; and optionally
(b) blending the dry powder with a lubricant and compressing the blend so formed into tablet cores; and optionally
(c) coating the tablet cores using a conventional pan coater.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with a high surface area excluding lactose such as SMCC, especially Prosolv SMCC®, a brittle filler with a low surface acidity such as dibasic calcium phosphate anhydrous milled grade, and optionally other excipients, to produce a homogenous mix; and optionally
(b) blending the dry powder with a lubricant such as magnesium stearate and compressing the blend so formed into tablet cores; and optionally
(c) coating the tablet cores using a conventional pan coater.

The film coat may be applied by spraying an aqueous suspension of the coating ingredients onto the tablet cores.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with an open porous structure excluding lactose, a brittle filler with a low surface acidity, optionally a secondary plastic filler and optionally other excipients, to produce a homogenous mix; and optionally
(b) blending the dry powder with a lubricant and compressing the blend so formed into tablet cores; and optionally
(c) coating the tablet cores using a conventional pan coater.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with an open porous structure excluding lactose such as Parteck M™ mannitol, a brittle filler with a low surface acidity such as dibasic calcium phosphate anhydrous milled grade, a secondary plastic filler such as microcrystalline cellulose and optionally other excipients, to produce a homogenous mix; and optionally
(b) blending the dry powder with a lubricant such as magnesium stearate and compressing the blend so formed into tablet cores; and optionally

(c) coating the tablet cores using a conventional pan coater.

The film coat may be applied by spraying an aqueous suspension of the coating ingredients onto the tablet cores.
According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with a high surface area excluding lactose, a brittle filler with a low surface acidity, optionally a secondary plastic filler and optionally other excipients to produce a homogenous mix;
(b) passing the homogeneous mix through a compactor to produce dry granules;
(c) adding further brittle filler with a low surface acidity and mixing the mixture;
(d) blending the dry granules so formed with a lubricant.

The powder may be formed into granules by a dry granulation technique, e.g. roller compaction.
The resultant granules may be compressed into tablet cores, which can then, if desired, be coated using a conventional pan coater. The film coat may be applied by spraying an aqueous suspension of the coating ingredients onto the tablet cores.
According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with a high surface area excluding lactose such as SMCC, especially Prosolv SMCC®, a brittle filler with a low surface acidity such as dibasic calcium phosphate anhydrous milled grade, and optionally other excipients to produce a homogenous mix;
(b) passing the homogeneous mix through a compactor to produce dry granules;
(c) adding further brittle filler with a low surface acidity such as dibasic calcium phosphate anhydrous milled grade and mixing the mixture;
(d) blending the dry granules so formed with a lubricant such as magnesium stearate.

The powder may be formed into granules by a dry granulation technique, e.g. roller compaction.
The resultant granules may be compressed into tablet cores, which can then, if desired, be coated using a conventional pan coater. The film coat may be applied by spraying an aqueous suspension of the coating ingredients onto the tablet cores.
According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with an open porous structure excluding lactose, a brittle filler with a low surface acidity, optionally a secondary plastic filler and optionally other excipients to produce a homogenous mix;
(b) passing the homogeneous mix through a compactor to produce dry granules;
(c) adding further brittle filler with a low surface acidity and mixing the mixture;
(d) blending the dry granules so formed with a lubricant.

The powder may be formed into granules by a dry granulation technique, e.g. roller compaction.
The resultant granules may be compressed into tablet cores, which can then, if desired, be coated using a conventional pan coater. The film coat may be applied by spraying an aqueous suspension of the coating ingredients onto the tablet cores.
According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with an open porous structure excluding lactose such as Parteck M™ mannitol, a brittle filler with a low surface acidity such as dibasic calcium phosphate anhydrous milled grade, a secondary plastic filler such as microcrystalline cellulose and optionally other excipients to produce a homogenous mix;
(b) passing the homogeneous mix through a compactor to produce dry granules;
(c) adding further brittle filler with a low surface acidity such as dibasic calcium phosphate anhydrous milled grade and mixing the mixture;
(d) blending the dry granules so formed with a lubricant such as magnesium stearate.

The powder may be formed into granules by a dry granulation technique, e.g. roller compaction.

The resultant granules may be compressed into tablet cores, which can then, if desired, be coated using a conventional pan coater. The film coat may be applied by spraying an aqueous suspension of the coating ingredients onto the tablet cores.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with a high surface area excluding lactose, a brittle filler with a low surface acidity, optionally a secondary plastic filler, and optionally other excipients to produce a homogeneous mix;
(b) adding a liquid binder to the powders with mixing until a wet mass is obtained;
(c) passing the wet granules through a screen to remove large particles;
(d) drying the mixture;
(e) passing the dried granules so formed through a further screen and blending the mixture with a lubricant.

The resultant granules may be compressed into tablet cores, which can then, if desired, be coated using a conventional pan coater. The film coat may be applied by spraying an aqueous suspension of the coating ingredients onto the tablet cores.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with a high surface area excluding lactose such as SMCC especially Prosolv SMCC®, a brittle filler with a low surface acidity such as dibasic calcium phosphate anhydrous milled grade, and optionally other excipients to produce a homogeneous mix;
(b) adding a liquid binder such as povidone to the powders with mixing until a wet mass is obtained;
(c) passing the wet granules through a screen to remove large particles;
(d) drying the mixture;
(e) passing the dried granules so formed through a further screen and blending the mixture with a lubricant such as magnesium stearate.

The resultant granules may be compressed into tablet cores, which can then, if desired, be coated using a conventional pan coater. The film coat may be applied by spraying an aqueous suspension of the coating ingredients onto the tablet cores.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with an open porous structure excluding lactose, a brittle filler with a low surface acidity, optionally a secondary plastic filler, and optionally other excipients to produce a homogeneous mix;
(b) adding a liquid binder to the powders with mixing until a wet mass is obtained;
(c) passing the wet granules through a screen to remove large particles;
(d) drying the mixture;
(e) passing the dried granules so formed through a further screen and blending the mixture with a lubricant.

The resultant granules may be compressed into tablet cores, which can then, if desired, be coated using a conventional pan coater. The film coat may be applied by spraying an aqueous suspension of the coating ingredients onto the tablet cores.

According to the present invention there is provided a process for the manufacture of a pharmaceutical composition comprising AZD2171 or a pharmaceutically acceptable salt thereof, comprising:

(a) mixing AZD2171 or a pharmaceutically acceptable salt thereof, especially AZD2171 maleate, a plastic filler with an open porous structure excluding lactose such as Parteck M™ mannitol, a brittle filler with a low surface acidity such as dibasic calcium phosphate anhydrous milled grade, a secondary plastic filler such as microcrystalline cellulose, and optionally other excipients to produce a homogeneous mix;
(b) adding a liquid binder such as povidone to the powders with mixing until a wet mass is obtained;
(c) passing the wet granules through a screen to remove large particles;
(d) drying the mixture;

(e) passing the dried granules so formed through a further screen and blending the mixture with a lubricant such as magnesium stearate.

The resultant granules may be compressed into tablet cores, which can then, if desired, be coated using a conventional pan coater. The film coat may be applied by spraying an aqueous suspension of the coating ingredients onto the tablet cores.

The formulated pharmaceutical compositions of the present invention may be made into tablets or granules or capsules and may be tested using the methods detailed below.

**Dissolution test method**

The dissolution of AZD2171 maleate tablets was tested using the method described in Table 1. A tentative evaluation criterion Q=75% at 45 minutes, wherein Q is the percentage of AZD2171 released at a given time, was applied.

**Table 1**

| | |
|---|---|
| Apparatus | : USP equipment - Teflon coated paddles, clear glass dissolution pots, six position minimum. United States Pharmacopoeia Apparatus 2 (paddle). |
| Medium volume | : 900 ml |
| Dissolution medium | : pH 4.5 (± 0.05) Buffer |
| Stirring speed | : 100 rpm |
| Temperature | : 37.0°C ± 0.5°C |
| Sampling volume | : 20 ml |
| Sampling times | : 15, 30 and 45 minutes |

**Dissolution medium preparation**

A pH 4.5 buffer was prepared, for example by dissolving 28.60 mL of glacial acetic acid in 9.9 litres of water. The solution was adjusted to pH 4.5 (± 0.05) with sodium hydroxide solution (5M) and made up to 10 litres with water.

**Disintegration Test Method**

The disintegration time of 6 individual tablets was determined, as described in Test A, in the European Pharmacopoeia (Ph Eur 2002), using water as the immersion fluid and omitting the discs. The time (minutes) at which all 6 tablets disintegrated was recorded.

Disintegration is considered to be achieved when:

(i) no residue remains on the screen, or
(ii) if there is a residue, it consists of a soft mass having no palpably firm, unmoistened core, or
(iii) only fragments of coating remain.

The tablets pass the test if all six have disintegrated within 30 minutes.

If any of the tablets have not disintegrated within 30 minutes, the test is repeated on a further six tablets using 0.1M HCl as disintegration medium.

**Yield Pressure Test Method**

The yield pressure of a material can be determined by compressing it in a compaction simulator using a simple uniaxial saw tooth displacement/time profile and measuring the corresponding forces. The compaction simulator is fitted with 10mm flat-faced punches and the material is compressed to zero porosity at a specific punch velocity. The force/ displacement data generated are manipulated to produce a Heckel plot and the yield pressure is equivalent to the reciprocal of the straight portion of the Heckel plot. This test can be performed at different punch velocities to determine the effect of scale-up on the deformation characteristics of the material.

Heckel equation

$$\ln\left(\frac{1}{1-D}\right) = KP + A$$

where K and A are constants obtained from the slope and intercept of the plot D is the relative density at pressure P.

(Ref: Roberts, R. J. and Rowe, R.C., The effect of punch velocity on the compaction of a variety of materials. J. Pharm. Pharmacol., 37 (1985) 377-384)

**Strain Rate Sensitivity (SRS)**

The strain rate sensitivity of a material can be calculated using the mean yield pressures (P*y*) calculated for a material at fast (300 mm $s^{-1}$) and slow (0.033 mm $s^{-1}$) punch velocities.

$$\mathrm{SRS} = \frac{Py_{300} - Py_{0.033}}{Py_{0.033}} \times 100$$

(Ref: Rowe, R. C., Roberts, R.J., Chapter 1, pg 34 in Advances in Pharmaceutical Sciences. Eds Ganderton, Jones, McGinity. Vol. 7 1995)

**Hardness Test Procedure (tablets)**

The hardness of 5 tablets every 30 minutes was tested as part of the in-process checks during tablet manufacture using a Schleuniger Hardness Tester Model 6D or equivalent. The hardness of each tablet was measured along its diameter. The average 'hardness' is reported in kiloponds (kp).

**Friability Test Method**

Twenty tablets were accurately weighed and placed in a rotating drum (Copley TA-10 or equivalent). The drum was rotated 100 times and the tablets removed. Loose dust was removed from the tablets and the tablets re-weighed. The friability is expressed as the loss of mass and it is calculated as a percentage of the initial mass.

**Methodology for the determination of surface pH of a brittle filler such as anhydrous dibasic calcium phosphate**

An estimation of the surface pH is obtained by measuring the pH of a high concentration aqueous slurry of the sample. A high concentration is not less than 1g/ml. A high concentration slurry in carbon dioxide-free water is prepared in a polytetrafluoroethylene (PTFE) container and is mixed by agitation for 2 minutes. The slurry is then degassed using nitrogen for 2 minutes before analysis. The pH is measured using a suitable pH probe, noting the maximum pH after insertion into the slurry.

The pharmaceutical compositions of the present invention are preferably formulated into tablets but may be made into another form: suitable for oral administration, (for example pellets, granules, lozenges, hard or soft capsules, dispersible powders or granules); or for vaginal or rectal administration (for example as a pessary or a suppository).

As stated above the size of the dose of AZD2171 required for the therapeutic or prophylactic treatment of a particular disease state will necessarily be varied depending on the host treated, the route of administration and the severity of the illness being treated. Preferably a daily dose in the range of 0.03-0.5mg/kg is employed. However the daily dose will necessarily be varied depending upon the host treated, the particular route of administration, and the severity of the illness being treated. Accordingly the optimum dosage may be determined by the practitioner who is treating any particular patient.

According to a further aspect of the present invention there is provided a pharmaceutical composition comprising AZD2171 or a pharmaceutically acceptable salt thereof according to the present invention as described hereinbefore, for use in a method of treatment of the human or animal body by therapy.

Pharmaceutical compositions of the present invention inhibit VEGF receptor tyrosine kinase activity and are therefore of interest for their antiangiogenic effects and/or their ability to cause a reduction in vascular permeability.

A further feature of the present invention is a pharmaceutical composition comprising AZD2171 or a pharmaceutically acceptable salt thereof according to the present invention as described hereinbefore, for use as a medicament, conveniently a pharmaceutical composition comprising AZD2171 or a pharmaceutically acceptable salt thereof according

to the present invention as described hereinbefore, for use as a medicament for producing an antiangiogenic and/or vascular permeability reducing effect in a warm-blooded animal such as a human being.

Thus according to a further aspect of the present invention there is provided the use of a pharmaceutical composition comprising AZD2171 or a pharmaceutically acceptable salt thereof according to the present invention as described hereinbefore in the manufacture of a medicament for use in the production of an antiangiogenic and/or vascular permeability reducing effect in a warm-blooded animal such as a human being.

According to a further feature of the present invention there is provided a method for producing an antiangiogenic and/or vascular permeability reducing effect in a warm-blooded animal, such as a human being, in need of such treatment which comprises administering to said animal an effective amount of a pharmaceutical composition comprising AZD2171 or a pharmaceutically acceptable salt thereof according to the present invention as described hereinbefore.

As stated above the pharmaceutical compositions of the present invention as defined herein are of interest for their antiangiogenic and/or vascular permeability effects. Angiogenesis and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, asthma, lymphoedema, endometriosis, dysfunctional uterine bleeding and ocular diseases with retinal vessel proliferation including age-related macular degeneration. Pharmaceutical compositions of the present invention are expected to be particularly useful in the prophylaxis and treatment of diseases such as cancer and Kaposi's sarcoma. In particular pharmaceutical compositions of the invention are expected to slow advantageously the growth of primary and recurrent solid tumours of, for example, the colon, pancreas, brain, bladder, liver, breast, prostate, lungs, soft tissues (for example soft tissue sarcoma) and skin. More especially pharmaceutical compositions of the present invention are expected to slow advantageously the growth of tumours in colorectal cancer and in lung cancer, for example mesothelioma, small cell lung cancer (SCLC) and non-small cell lung cancer (NSCLC). More particularly pharmaceutical compositions of the invention are expected to inhibit any form of cancer associated with VEGF including leukaemia, multiple myeloma and lymphoma and also, for example, to inhibit the growth of those primary and recurrent solid tumours which are associated with VEGF, especially those tumours which are significantly dependent on VEGF for their growth and spread, including for example, certain tumours of the colon (including rectum), pancreas, brain, bladder, breast, prostate, lung, vulva, skin and particularly NSCLC.

In another aspect of the present invention the pharmaceutical compositions of the present invention as defined herein are expected to inhibit the growth of those primary and recurrent solid tumours which are associated with VEGF especially those tumours which are significantly dependent on VEGF for their growth and spread.

The pharmaceutical compositions of the present invention as defined herein may be administered as a sole therapy or may involve, in addition to a composition of the present invention, one or more other substances and/or treatments. Such conjoint treatment may be achieved by way of the simultaneous, sequential or separate administration of the individual components of the treatment. In the field of medical oncology it is normal practice to use a combination of different forms of treatment to treat each patient with cancer. In medical oncology the other component(s) of such conjoint treatment in addition to the antiangiogenic and/or vascular permeability reducing treatment defined hereinbefore may be: surgery, radiotherapy or chemotherapy.

Surgery may comprise the step of partial or complete tumour resection, prior to, during or after the administration of a pharmaceutical composition as described herein.

Other chemotherapeutic agents for optional use with a pharmaceutical composition of the present invention as defined herein include those described in WO 00/47212 and WO 05/061488 which are both incorporated herein by reference. Such chemotherapy may cover five main categories of therapeutic agent:

(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology; and other categories of agent are:
(vi) antisense therapies;
(vii) gene therapy approaches; and
(ix) immunotherapy approaches.

The invention is illustrated below by the following non-limiting examples:

## Example 1

**Composition of AZD2171 0.5mg coated tablet (6.0mm normal concave (N/C) round)**

| Ingredient | mg/tab | Function |
|---|---|---|
| Tablet Core | | |
| AZD2171 maleate | 0.63 | Active agent |
| Mannitol[1] | 94.37 | Plastic filler |
| Sodium starch glycolate[2] | 4.00 | Disintegrant |
| Magnesium stearate[3] | 1.00 | Lubricant |
| **Total** | **100 mg** | |
| Tablet Coating | | |
| Hypromellose[4] | 1.53 | Film-forming agent |
| Macrogol 300[5] | 0.30 | Plasticiser |
| Red iron oxide[6] | 0.18 | Pigment |
| Yellow iron oxide[6] | 0.18 | Pigment |
| Titanium dioxide[7] Solvent | 0.10 | Opacifier |
| **Total** | **2.29 mg** | |
| **Nominal coated tablet weight** | **102.29 mg** | |

Footnotes: The following excipients were used in Example 1:
[1] Parteck M™ mannitol (Merck Chemicals Ltd.,Poole, UK)
[2] Glycolys™ sodium starch glycolate (Roquette Frères 62080, Lestrem, France).
[3] Magnesium Stearate (Mallinckrodt, St Louis, Missouri, USA).
[4] Pharmacoat™ 606, Hydroxypropyl methylcellulose Grade 2910, 6cP dynamic viscosity (measured at 2%w/v in water at 20˚C) (ex Shin Etsu).
[5] Polyethylene glycol 300, Reagent Chemical Services Ltd (Runcorn UK).
[6] Red and yellow iron oxides and a portion of the hydroxypropyl methylcellulose were provided in Orange Speedpaste (Ansteads Ltd., UK)
[7] Titanium dioxide and a portion of the hydroxypropyl methylcellulose were provided in White Speedpaste, (Ansteads Ltd., UK)

The formulation described as Example 1 was prepared by conventional direct compression and film coating processes.

The AZD2171 maleate and the mannitol were sieved into a bowl in the following order: approximately ¼ mannitol, AZD2171 maleate, approximately ¼ mannitol and then mixed together in a planetary mixer for 10 minutes. The remaining mannitol and the sodium starch glycolate were then added to the bowl and the mixture was mixed for a further 10 minutes. The magnesium stearate was then added through a sieve and the mixture was mixed for a further 5 minutes. The resultant mixture was then compressed into tablet cores and coated using a conventional pan coater. The film coat was applied by spraying an aqueous suspension of hypromellose (hydroxypropyl methylcellulose), polyethylene glycol 300, red iron oxide, yellow iron oxide, and titanium dioxide onto the tablet cores.

Tablets of Example 1 formulation were compressed using a single punch F-press and tested using methods described previously.

The hardness of tablet cores compressed at 200 MPa (megapascals) was 11kp (kiloponds) and the mean friability of the tablet cores was 0.29%. The mean disintegration time for tablet cores with a hardness of 11kp was 2 minutes. In the dissolution test using coated tablets more than 75% of the AZD2171 was observed to dissolve within 45 minutes at pH 4.5.

**Example 2**

**Composition of AZD2171 30mg coated tablet (9.0 mm N/C round)**

| **Ingredient** | **mg/tab** | **Function** |
|---|---|---|
| Tablet core | | |
| AZD2171 maleate | 37.80 | Active agent |
| Mannitol[1] | 200.70 | Plastic filler |
| Dibasic calcium phosphate anhydrous milled grade[2] | 45.00 | Brittle filler |
| Sodium starch glycolate[3] | 12.00 | Disintegrant |
| Magnesium stearate[4] | 4.50 | Lubricant |
| **Total** | **300 mg** | |
| Tablet coating[5] | | |
| Hypromellose | 6.759 | Film-forming agent |
| Macrogol 400 | 0.676 | Plasticiser |
| Red iron oxide | 0.030 | Pigment |
| Yellow iron oxide | 0.122 | Pigment |
| Black iron oxide | 0.008 | Pigment |
| Titanium Dioxide | 3.220 | Opacifier |
| Purified water | | Solvent |
| **Total** | **10.815 mg** | |
| **Nominal coated tablet weight** | **310.815 mg** | |

Footnotes: The following excipients were used in Example 2:
[1] Parteck M™ mannitol (Merck Chemicals Ltd.,Poole, UK)
[2] Calipharm A™ dibasic calcium phosphate anhydrous milled grade (Rhodia Inc, Etoile Part-Dieu, France)
[3] Glycolys™ sodium starch glycolate (Roquette Frères 62080, Lestrem, France).
[4] Magnesium Stearate (Mallinckrodt, St Louis, Missouri, USA).
[5] Coating supplied as Opadry Beige 03B27164, Colorcon Ltd, Dartford, Kent, UK

The formulation described as Example 2 was prepared by conventional dry granulation, compression and film coating processes.

The AZD2171 maleate and the mannitol were sieved into a bowl in the following order: approximately ¼ mannitol, AZD2171 maleate, approximately ¼ mannitol and then mixed together in a planetary mixer for 10 minutes. The remaining mannitol, one sixth of the dibasic calcium phosphate and the sodium starch glycolate were then added to the bowl and the mixture was mixed for a further 10 minutes. One third of the magnesium stearate was then added through a sieve and the mixture was mixed for a further 2 minutes. The resultant mixture was then passed through a roller compactor to produce the dry granules. The additional dibasic calcium phosphate was then added to the granules and the mixture was mixed for a further 5 minutes. The resultant granules were then added to a blender, with the additional magnesium stearate sieved in. The mixture was then blended for 5 minutes. The granules were then compressed into tablet cores and coated using a conventional pan coater. The film coat was applied by spraying an aqueous suspension of hypromellose, Macrogol 400, red iron oxide, yellow iron oxide, black iron oxide and titanium oxide onto the tablet cores.

Tablets of Example 2 formulation were compressed using a high-speed rotary press and tested using methods described previously.

The hardness of tablet cores compressed at 200 MPa was 12 kp and the mean friability of the tablet cores was 0.15%. The mean disintegration time for tablet cores with a hardness of 12 kp was less than 1 minute. In the dissolution test using coated tablets more than 75% of the AZD2171 was observed to dissolve within 45 minutes at pH 4.5.

**Example 3**

**Composition of AZD2171 30mg coated tablet (9.0 mm N/C round)**

| Ingredient | mg/tab | Function |
|---|---|---|
| Tablet core | | |
| AZD2171 maleate | 37.8 | Active agent |
| Mannitol[1] | 163.2 | Plastic filler |
| Dibasic calcium phosphate anydrous milled grade[2] | 45.0 | Brittle filler |
| Microcrystalline cellulose[3] | 37.5 | Secondary plastic filler |
| Sodium starch glycolate[4] | 12.0 | Disintegrant |
| Magnesium stearate[5] | 4.5 | Lubricant |
| **Total** | **300 mg** | |
| Tablet coatingb[6] | | |
| Hypromellose | 6.759 | Film-forming agent |
| Macrogol 400 | 0.676 | Plasticiser |
| Red iron oxide | 0.030 | Pigment |
| Yellow iron oxide | 0.122 | Pigment |
| Black iron oxide | 0.008 | Pigment |
| Titanium Dioxide | 3.220 | Opacifier |
| Purified water | | Solvent |
| **Total** | **10.815 mg** | |
| **Nominal coated tablet weight** | **310.815 mg** | |

Footnotes: The following excipients were used in Example 3:
[1] Parteck M™ mannitol (Merck Chemicals Ltd.,Poole, UK)
[2] Calipharm A™ dibasic calcium phosphate anhydrous milled grade (Rhodia Inc,Etoile Part-Dieu, France)
[3] Avicel™ microcrystalline cellulose (ex. FMC International, Philadelphia, Pennsylvania, USA).
[4] Glycolys™ sodium starch glycolate (Roquette Frères 62080, Lestrem, France).
[5] Magnesium Stearate ex. Mallinckrodt, St Louis, Missouri, USA.
[6] Coating supplied as Opadry Beige 03B27164, Colorcon Ltd, Dartford, Kent, UK.

The formulation described as Example 3 was prepared by conventional dry granulation, compression and film coating processes.

The AZD2171 maleate and the mannitol were sieved into a bowl in the following order: approximately ¼ mannitol, AZD2171 maleate, approximately ¼ mannitol and then mixed together in a planetary mixer for 10 minutes. The remaining mannitol, one third of the dibasic calcium phosphate and the sodium starch glycolate were then added to the bowl and the mixture was mixed for a further 10 minutes. One sixth of the magnesium stearate was then added through a sieve and the mixture was mixed for a further 2 minutes. The resultant mixture was then passed through a roller compactor to produce the dry granules. The additional dibasic calcium phosphate was then added to the granules and the mixture was mixed for a further 5 minutes. The resultant granules were then added to a blender, with the additional magnesium stearate sieved in. The mixture was then blended for 5 minutes. The granules were then compressed into tablet cores and coated using a conventional pan coater. The film coat was applied by spraying an aqueous suspension of hypromellose, Macrogol 400, red iron oxide, yellow iron oxide, black iron oxide and titanium oxide onto the tablet cores.

Tablets of Example 3 formulation were compressed using a high-speed rotary press and tested using methods described previously.

The hardness of tablet cores compressed at 200 MPa was 12 kp and the mean friability of the tablet cores was 0.08%. No tablet defects (including capping) were observed during the manufacture of these tablets. The mean disintegration time for tablet cores with a hardness of 12 kp was 2.5 minutes. In the dissolution test using coated tablets more than 75% of the AZD2171 was observed to dissolve within 45 minutes at pH 4.5.

<u>Example 4</u>

**Composition of AZD2171 45mg coated tablet (8.0 mm N/C round)**

| Ingredient | mg/tab | Function |
|---|---|---|
| Tablet Core | | |
| AZD2171 maleate | 56.70 | Active agent |
| Mannitol[1] | 77.50 | Plastic filler |
| Dibasic calcium phosphate anhydrous milled grade[2] | 30.00 | Brittle filler |
| Microcrystalline cellulose[3] | 20.80 | Secondary plastic filler |
| Sodium starch glycolate[4] | 8.00 | Disintegrant |
| Povidone[5] | 4.00 | Binder |
| Magnesium stearate[6] | 3.00 | Lubricant |
| **Total** | **200 mg** | |
| Tablet coating[7] | | |
| Hypromellose | 4.506 | Film-forming agent |
| Macrogol 400 | 0.451 | Plasticiser |
| Red iron oxide | 0.020 | Pigment |
| Yellow iron oxide | 0.081 | Pigment |
| Black iron oxide | 0.005 | Pigment |
| Titanium Dioxide | 2.147 | Opacifier |
| Purified water | | Solvent |
| **Total** | **7.210 mg** | |
| **Nominal coated tablet weight** | **207.210 mg** | |

Footnotes: The following excipients were used in Example 4:
[1] Parteck M™ mannitol (Merck Chemicals Ltd.,Poole, UK)
[2] Calipharm A™ dibasic calcium phosphate anhydrous milled grade (Rhodia Inc,Etoile Part-Dieu, France)
[3] Avicel™ microcrystalline cellulose (FMC International, Philadelphia, Pennsylvania, USA).
[4] Glycolys™ sodium starch glycolate (Roquette Frères 62080, Lestrem, France).
[5] Plasdone™ povidone K29-K32 (International Speciality Products, Wayne, New Jersey, USA).
[6] Magnesium Stearate (Mallinckrodt, St Louis, Missouri, USA).
[7] Coating supplied as Opadry Beige 03B27164, (Colorcon Ltd, Dartford, Kent, UK).

The formulation described as Example 4 was prepared by conventional wet granulation, compression and film coating processes.

The AZD2171 maleate, the mannitol, the dibasic calcium phosphate, the microcrystalline cellulose and the sodium starch glycolate were mixed together in a high shear granulator for 10 minutes to produce an homogenous mix. A 13.3% w/v solution of povidone was then added to the powders with 2 minutes 30 seconds total mixing time to produce a wet mass. The wet granules were passed through a screen to remove large particles then dried. The dried particles were then passed through a further screen and blended with 1.5% w/w pre-milled magnesium stearate for 5 minutes. The resultant blend was compressed into tablet cores, which were then coated using a conventional pan coater. The film coat was applied by spraying an aqueous suspension of hypromellose, Macrogol 400, red iron oxide, yellow iron oxide, black iron oxide and titanium oxide onto the tablet cores.

Tablets of Example 4 formulation were compressed using a high-speed rotary press and tested using methods described previously.

The hardness of tablet cores compressed at 200 MPa was 17 kp and the mean friability of the tablet cores was 0.17%. No tablet defects (including capping) were observed during the manufacture of these tablets. The mean disintegration time of tablet cores with a hardness of 17 kp was 4 minutes 9 seconds. In the dissolution test using coated tablets more than 75% of the AZD2171 was observed to dissolve within 45 minutes at pH 4.5.

**Example 5**

**Composition of AZD2171 30mg tablet cores (9.0 mm N/C round)**

| Ingredient | mg/tab | Function |
|---|---|---|
| Tablet core | | |
| AZD2171 maleate | 37.8 | Active agent |
| Silicified Microcrystalline cellulose[1] | 245.7 | Plastic filler |
| Sodium starch glycolate[2] | 12.0 | Disintegrant |
| Magnesium stearate[3] | 4.5 | Lubricant |
| **Total** | **300 mg** | |

Footnotes: The following excipients were used in Example 5:
[1] Prosolv SMCC® 90 (JRS PHARMA GmbH+Co.KG, Rosenberg, Germany).
[2] Glycolys™ sodium starch glycolate (Roquette Freres 62080, Lestrem, France).
[3] Magnesium Stearate (Mallinckrodt, St Louis, Missouri, USA).

The formulation described as Example 5 was prepared by conventional direct compression.

The AZD2171 maleate and the Prosolv SMCC® 90 were sieved into a bowl in the following order: approximately ¼ silicified microcrystalline cellulose (SMCC), AZD2171 maleate, approximately ¼ SMCC and then mixed together in a planetary mixer for 10 minutes. The remaining SMCC and the sodium starch glycolate were then added to the bowl and the mixture was mixed for a further 10 minutes. The magnesium stearate was then added through a sieve and the mixture was mixed for a further 5 minutes. The resultant mixture was then compressed into tablet cores. Tablets of Example 5 formulation were compressed using a single punch F-press and tested using methods described previously.

The hardness of tablet cores compressed at 100 MPa (megapascals) was 11kp (kiloponds). Capping was seen at compaction pressures greater than about 150 MPa. The mean disintegration time for tablet cores with a hardness of 11kp was 16 seconds.

## Example 6

**Composition of AZD2171 30mg tablet cores (9.0 mm N/C round)**

| Ingredient | mg/tab | Function |
|---|---|---|
| Tablet core | | |
| AZD2171 maleate | 37.8 | Active agent |
| Silicified Microcrystalline cellulose[1] | 200.7 | Plastic filler |
| Dibasic calcium phosphate anydrous milled grade[2] | 45.0 | Brittle filler |
| Sodium starch glycolate[3] | 12.0 | Disintegrant |
| Magnesium stearate[4] | 4.5 | Lubricant |
| **Total** | **300 mg** | |

Footnotes: The following excipients were used in Example 6:
[1] Prosolv SMCC® 50 (JRS PHARMA GmbH+Co.KG, Rosenberg, Germany).
[2] Calipharm A™ dibasic calcium phosphate anhydrous milled grade (Rhodia Inc,Etoile Part-Dieu, France).
[3] Glycolys™ sodium starch glycolate (Roquette Frères 62080, Lestrem, France).
[4] Magnesium Stearate (Mallinckrodt, St Louis, Missouri, USA).

The formulation described as Example 6 was prepared by conventional direct compression.

The AZD2171 maleate and the Prosolv SMCC® 50 were sieved into a bowl in the following order: approximately ¼ silicified microcrystalline cellulose (SMCC), AZD2171 maleate, approximately ¼ SMCC and then mixed together in a planetary mixer for 10 minutes. The remaining SMCC, the dibasic calcium phosphate, anhydrous and the sodium starch glycolate were then added to the bowl and the mixture was mixed for a further 10 minutes. The magnesium

stearate was then added through a sieve and the mixture was mixed for a further 5 minutes. The resultant mixture was then compressed into tablet cores. Tablets of Example 6 formulation were compressed using a single punch F-press and tested using methods described previously.

The hardness of tablet cores compressed at 75 MPa (megapascals) was 11kp (kiloponds). No evidence of capping was observed during the manufacture of these tablets. The mean disintegration time for tablet cores with a hardness of 11kp was 12 seconds.

## Claims

1. A pharmaceutical composition comprising AZD2171 or a pharmaceutically acceptable salt thereof and a plastic filler with a high surface area, excluding lactose.

2. A pharmaceutical composition according to claim 1 wherein the plastic filler with a high surface area excluding lactose is silicified microcrystalline cellulose.

3. A pharmaceutical composition according to claim 2 wherein the plastic filler with a high surface area is Prosolv®.

4. A pharmaceutical composition according to claim 1 wherein the plastic filler with a high surface area is a plastic filler with an open porous structure excluding lactose.

5. A pharmaceutical composition according to claim 4 wherein the plastic filler with an open porous structure excluding lactose is Parteck M™ mannitol.

6. A pharmaceutical composition according to claim 1 comprising AZD2171 or a pharmaceutically acceptable salt thereof, a plastic filler with a high surface area excluding lactose and a brittle filler with a low surface acidity.

7. A pharmaceutical composition as claimed in claim 6 wherein AZD2171 is in the form of AZD2171 maleate, the plastic filler with a high surface area is silicified microcrystalline cellulose and the brittle filler with a low surface acidity is dibasic calcium phosphate anhydrous milled grade.

8. A pharmaceutical composition according to claim 4 comprising AZD2171 or a pharmaceutically acceptable salt thereof, a plastic filler with an open porous structure excluding lactose and a brittle filler with a low surface acidity.

9. A pharmaceutical composition as claimed in claim 8 wherein AZD2171 is in the form of AZD2171 maleate, the plastic filler with an open porous structure is Parteck M™ mannitol and the brittle filler with a low surface acidity is dibasic calcium phosphate anhydrous milled grade.

10. A pharmaceutical composition comprising AZD2171 or a pharmaceutically acceptable salt thereof and a brittle filler with a low surface acidity.

11. A pharmaceutical composition according to claim 10 wherein the brittle filler with a low surface acidity is dibasic calcium phosphate anhydrous milled grade.

12. A pharmaceutical composition according to claim 6 comprising AZD2171 or a pharmaceutically acceptable salt thereof, a plastic filler with a high surface area excluding lactose, a brittle filler with a low surface acidity and optionally a secondary plastic filler.

13. A pharmaceutical composition according to claim 8 comprising AZD2171 or a pharmaceutically acceptable salt thereof, a plastic filler with an open porous structure excluding lactose, a brittle filler with a low surface acidity and optionally a secondary plastic filler.

14. A pharmaceutical composition according to claim 13 comprising AZD2171 or a pharmaceutically acceptable salt thereof, a plastic filler with an open porous structure excluding lactose, a brittle filler with a low surface acidity and a secondary plastic filler.

15. A pharmaceutical composition according to any one of the preceding claims further comprising a disintegrant.

**16.** A pharmaceutical composition according to any one of the preceding claims further comprising a lubricant.

**17.** A pharmaceutical composition according to any one of the preceding claims further comprising a binder.

**18.** A pharmaceutical composition comprising:

(a) from 0.1 to 50 parts AZD2171 or a pharmaceutically acceptable salt thereof;
(b) from 15 to 95 parts of a plastic filler with a high surface area excluding lactose; and
(c) from 0 to 50 parts of a brittle filler with a low surface acidity;

wherein all parts are by weight and the sum of the parts (a)+(b)+(c)=100.

**19.** A pharmaceutical composition comprising:

(a) from 0.1 to 50 parts AZD2171 or a pharmaceutically acceptable salt thereof;
(b) from 15 to 95 parts of a plastic filler with a high surface area excluding lactose;
(c) from 0 to 50 parts of a brittle filler with a low surface acidity;
(d) from 0 to 50 parts of a secondary plastic filler;
(e) from 0.1 to 10 parts of a disintegrant; and
(f) from 0.01 to 8 parts of a lubricant;

wherein all parts are by weight and the sum of the parts (a)+(b)+(c)+(d)+(e)+(f) = 100.

**20.** A pharmaceutical composition comprising:

(a) from 0.1 to 50 parts AZD2171 or a pharmaceutically acceptable salt thereof;
(b) from 15 to 95 parts of a plastic filler with an open porous structure excluding lactose; and
(c) from 1 to 50 parts of a brittle filler with a low surface acidity;

wherein all parts are by weight and the sum of the parts (a)+(b)+(c)=100.

**21.** A pharmaceutical composition comprising:

(a) from 0.1 to 50 parts AZD2171 or a pharmaceutically acceptable salt thereof;
(b) from 15 to 95 parts of a plastic filler with an open porous structure excluding lactose;
(c) from 1 to 50 parts of a brittle filler with a low surface acidity;
(d) from 1 to 50 parts of a secondary plastic filler;
(e) from 0.1 to 10 parts of a disintegrant; and
(f) from 0.01 to 8 parts of a lubricant;

wherein all parts are by weight and the sum of the parts (a)+(b)+(c)+(d)+(e)+(f) = 100.

**22.** A pharmaceutical composition comprising a core that comprises a pharmaceutical composition according to any one of the preceding claims, and a coating.

**23.** A process for the manufacture of a pharmaceutical composition comprising AZD2171 or a pharmaceutically acceptable salt thereof, comprising:

(a) mixing AZD2171 or a pharmaceutically acceptable salt thereof, a plastic filler with a high surface area excluding lactose, a brittle filler with a low surface acidity, optionally a secondary plastic filler and optionally other excipients, to produce a homogenous mix; and optionally
(b) blending the dry powder with a lubricant and compressing the blend so formed into tablet cores; and optionally
(c) coating the tablet cores using a conventional pan coater.

**24.** A process for the manufacture of a pharmaceutical composition comprising AZD2171 or a pharmaceutically acceptable salt thereof, comprising:

(a) mixing AZD2171 or a pharmaceutically acceptable salt thereof, a plastic filler with a high surface area excluding lactose, a brittle filler with a low surface acidity, optionally a secondary plastic filler and optionally other excipients to produce a homogenous mix;
(b) passing the homogeneous mix through a compactor to produce dry granules;
(c) adding further brittle filler with a low surface acidity and mixing the mixture;
(d) blending the dry granules so formed with a lubricant.

**25.** A process for the manufacture of a pharmaceutical composition comprising AZD2171 or a pharmaceutically acceptable salt thereof, comprising:

(a) mixing AZD2171 or a pharmaceutically acceptable salt thereof, a plastic filler with a high surface area excluding lactose, a brittle filler with a low surface acidity, optionally a secondary plastic filler, and optionally other excipients to produce a homogeneous mix;
(b) adding a liquid binder to the powders with mixing until a wet mass is obtained;
(c) passing the wet granules through a screen to remove large particles;
(d) drying the mixture;
(e) passing the dried granules so formed through a further screen and blending the mixture with a lubricant.

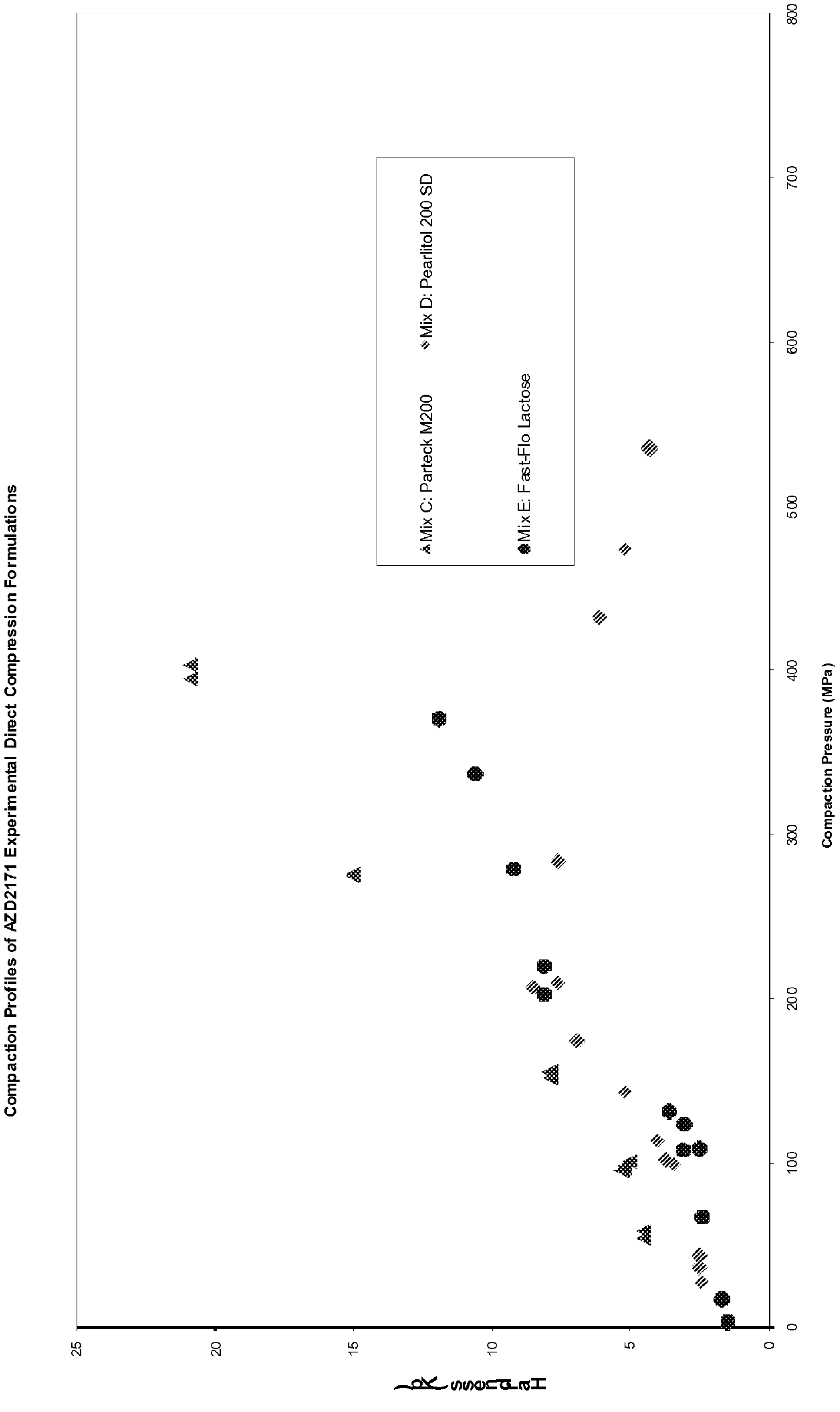
Compaction Profiles of AZD2171 Experimental Direct Compression Formulations
Mix C: Parteck M200
Mix D: Pearlitol 200 SD
Mix E: Fast-Flo Lactose
Compaction Pressure (MPa)
0
100
200
300
400
500
600
700
800
0
5
10
15
20
25

Figure 1

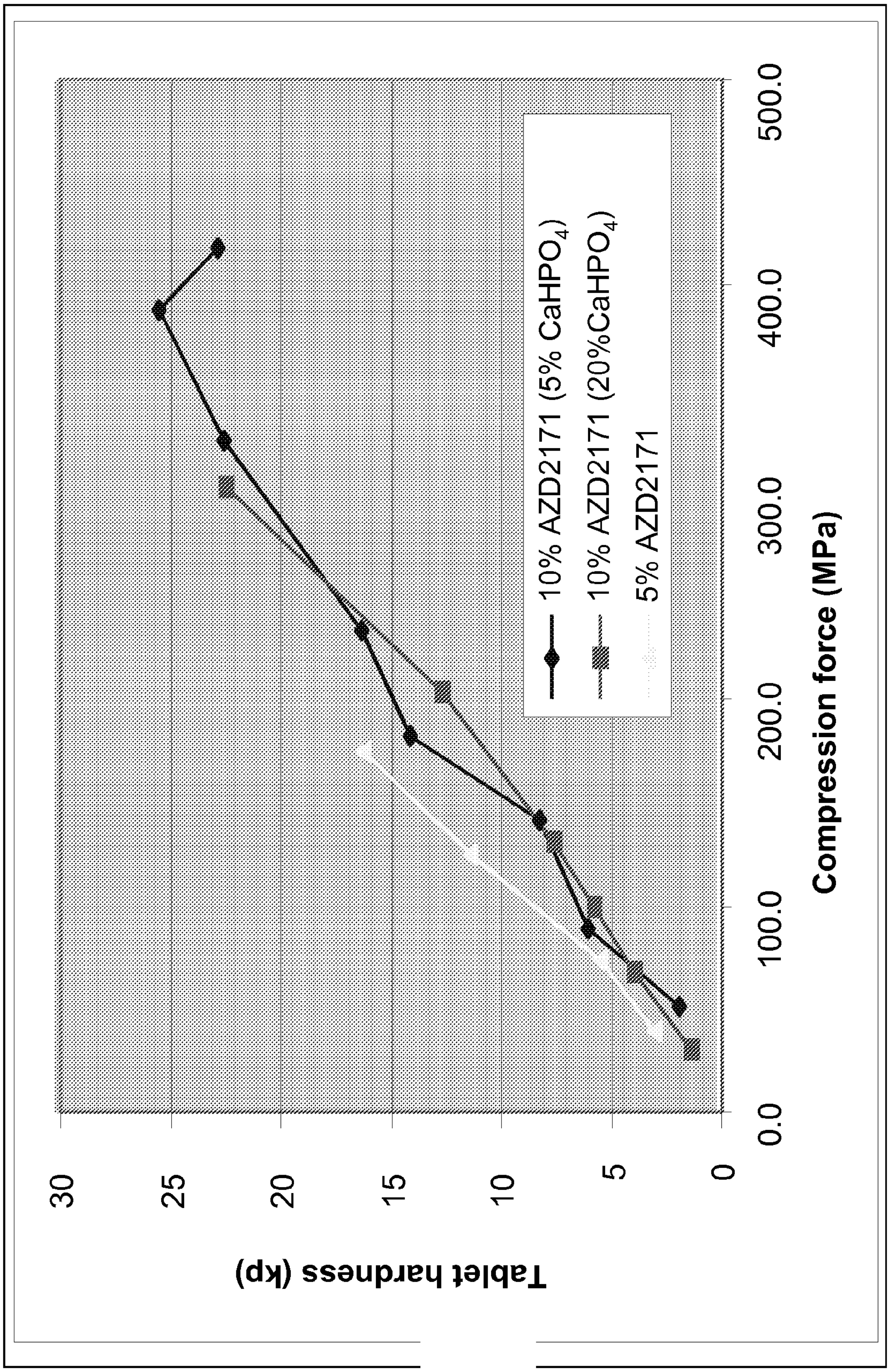
Tablet hardness (kp)
30
25
20
15
10
5
0
0.0
100.0
200.0
300.0
400.0
500.0
Compression force (MPa)
10% AZD2171 (5% $CaHPO_4$)
10% AZD2171 (20%$CaHPO_4$)
5% AZD2171

Figure 2

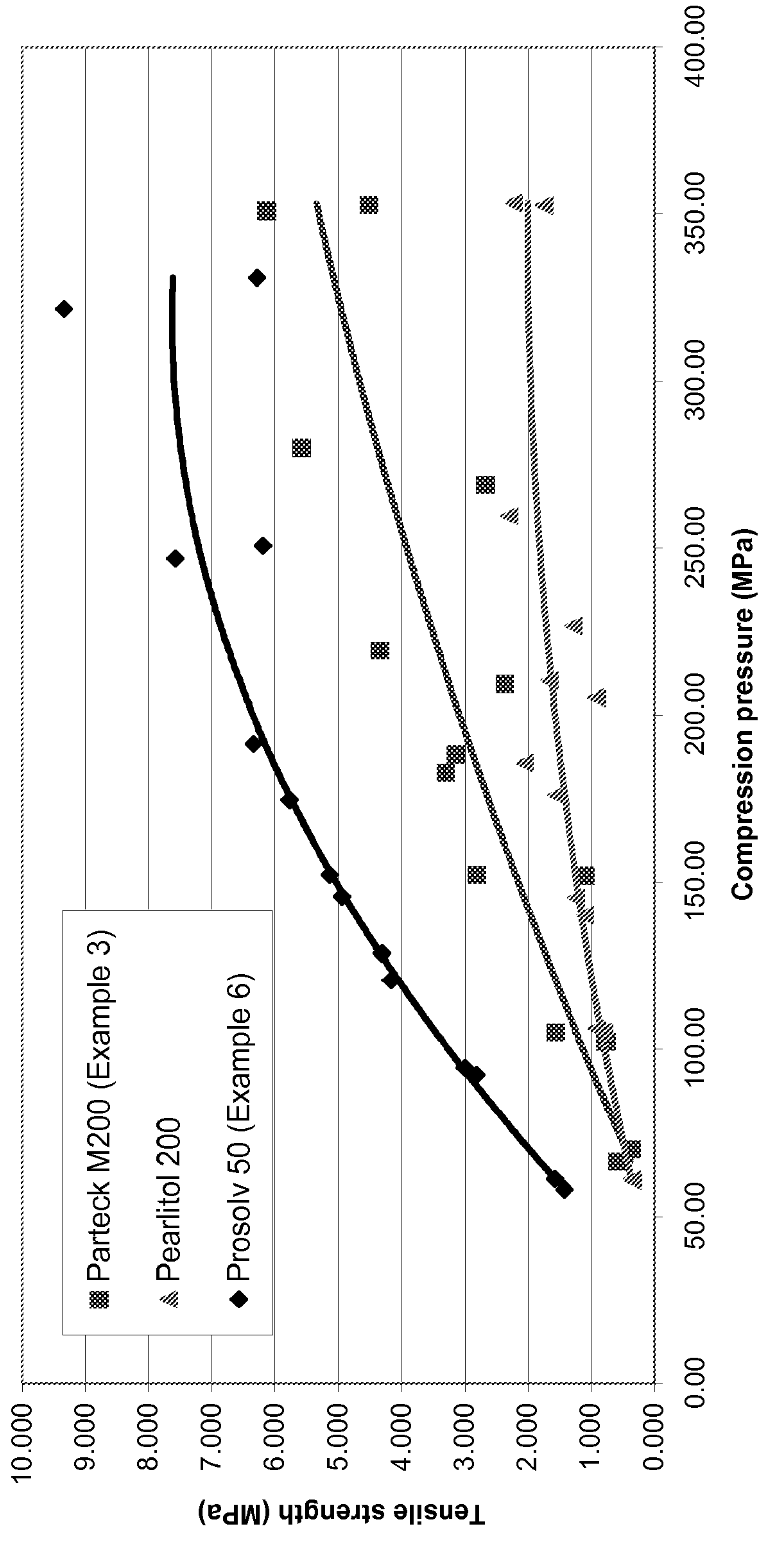
Tensile strength (MPa)
10.000
9.000
8.000
7.000
6.000
5.000
4.000
3.000
2.000
1.000
0.000
Compression pressure (MPa)
0.00
50.00
100.00
150.00
200.00
250.00
300.00
350.00
400.00
Parteck M200 (Example 3)
Pearlitol 200
Prosolv 50 (Example 6)

Figure 3

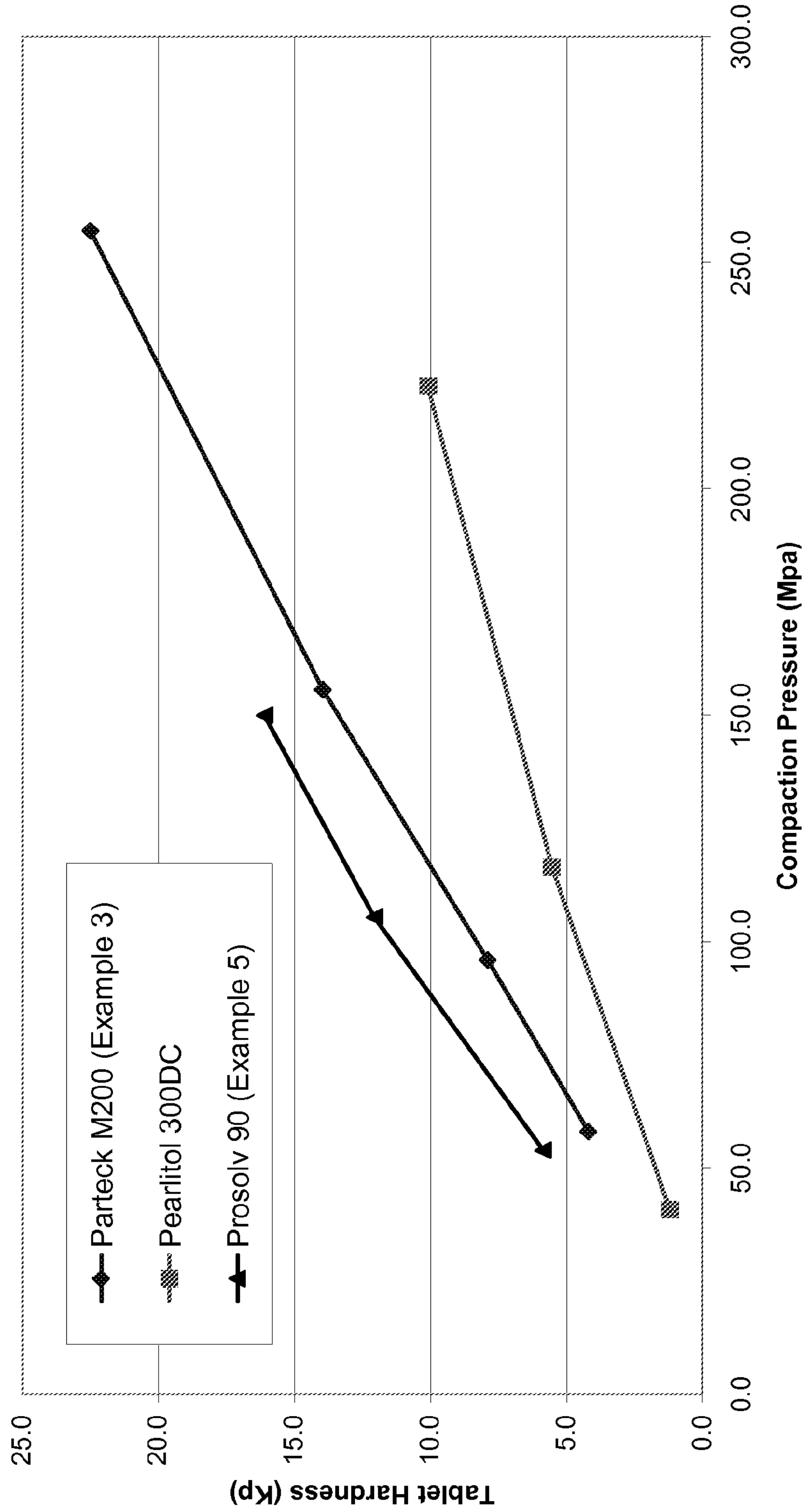
Tablet Hardness (Kp)
25.0
20.0
15.0
10.0
5.0
0.0
0.0
50.0
100.0
150.0
200.0
250.0
300.0
Compaction Pressure (Mpa)
Parteck M200 (Example 3)
Pearlitol 300DC
Prosolv 90 (Example 5)

Figure 4

Europäisches Patentamt
European Patent Office
Office européen des brevets

# EUROPEAN SEARCH REPORT

Application Number
EP 10 18 2729

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/064413 A (ASTRAZENECA R & D SODERTALJE [SE]; ASTRAZENECA UK LTD [GB]; HENNEQUIN) 7 August 2003 (2003-08-07) * page 160 - page 161; claims 20-22 * * examples (e), (f) and (g) on pages 160-161 * ----- | 1-25 | INV. A61K9/20 A61K31/517 |
| A | WO 2004/096224 A (BOEHRINGER INGELHEIM INT [DE]; BOEHRINGER INGELHEIM PHARMA [DE]; HILBE) 11 November 2004 (2004-11-11) * page 60, paragraph 2 * ----- | 1-25 | |
| A,D | WO 2005/061488 A (ASTRAZENECA AB [SE]; ASTRAZENECA UK LTD [GB]; MCCABE JAMES [GB]) 7 July 2005 (2005-07-07) * claims 14-21 * ----- | 1-25 | |
| Y | WO 00/47212 A1 (ASTRAZENECA UK LTD [GB]; ZENECA PHARMA SA [FR]; HENNEQUIN LAURENT FRAN) 17 August 2000 (2000-08-17) * claims 21,22; examples 240, 326 * ----- | 1-25 | |

TECHNICAL FIELDS SEARCHED (IPC)

A61K

The present search report has been drawn up for all claims

1

| Place of search | Date of completion of the search | Examiner |
|---|---|---|
| Munich | 27 December 2010 | Kardas-Llorens, Eyüp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 10 18 2729

This annex lists the patent family members relating to the patent documents cited in the above-mentioned European search report.
The members are as contained in the European Patent Office EDP file on
The European Patent Office is in no way liable for these particulars which are merely given for the purpose of information.

27-12-2010

| Patent document cited in search report | Publication date | Patent family member(s) | Publication date |
|---|---|---|---|
| WO 03064413 A | 07-08-2003 | AR 038476 A1 | 19-01-2005 |
| | | AU 2003202094 B2 | 08-10-2009 |
| | | BR 0307151 A | 07-12-2004 |
| | | CA 2473572 A1 | 07-08-2003 |
| | | CN 1625555 A | 08-06-2005 |
| | | CN 101607958 A | 23-12-2009 |
| | | EP 1474420 A1 | 10-11-2004 |
| | | HU 0402588 A2 | 30-05-2005 |
| | | IS 7362 A | 22-07-2004 |
| | | JP 2005522428 T | 28-07-2005 |
| | | JP 2010100642 A | 06-05-2010 |
| | | MX PA04007459 A | 08-09-2005 |
| | | NO 328731 B1 | 03-05-2010 |
| | | NZ 534171 A | 29-06-2007 |
| | | RU 2365588 C2 | 27-08-2009 |
| | | RU 2362774 C1 | 27-07-2009 |
| | | RU 2362775 C1 | 27-07-2009 |
| | | UA 81619 C2 | 25-01-2008 |
| | | US 2005085465 A1 | 21-04-2005 |
| | | US 2008027069 A1 | 31-01-2008 |
| | | US 2009156821 A1 | 18-06-2009 |
| | | ZA 200405908 A | 26-09-2005 |
| WO 2004096224 A | 11-11-2004 | AR 044114 A1 | 24-08-2005 |
| | | AU 2004233576 A1 | 11-11-2004 |
| | | AU 2010236075 A1 | 18-11-2010 |
| | | BR PI0409919 A | 25-04-2006 |
| | | CA 2523868 A1 | 11-11-2004 |
| | | CL 9002004 A1 | 04-03-2005 |
| | | CO 5640112 A2 | 31-05-2006 |
| | | EA 200900272 A1 | 30-10-2009 |
| | | EC SP056132 A | 01-03-2006 |
| | | JP 2006524634 T | 02-11-2006 |
| | | JP 2010202658 A | 16-09-2010 |
| | | KR 20060008945 A | 27-01-2006 |
| | | MX PA05011656 A | 15-12-2005 |
| | | NZ 543774 A | 30-10-2009 |
| | | UY 28287 A1 | 30-11-2004 |
| WO 2005061488 A | 07-07-2005 | AR 046993 A1 | 04-01-2006 |
| | | AT 471936 T | 15-07-2010 |
| | | AU 2004303590 A1 | 07-07-2005 |
| | | BR PI0417958 A | 27-03-2007 |
| | | CA 2548662 A1 | 07-07-2005 |
| | | CN 1898232 A | 17-01-2007 |
| | | DK 1699782 T3 | 30-08-2010 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 10 18 2729

This annex lists the patent family members relating to the patent documents cited in the above-mentioned European search report.
The members are as contained in the European Patent Office EDP file on
The European Patent Office is in no way liable for these particulars which are merely given for the purpose of information.

27-12-2010

| Patent document cited in search report | Publication date | Patent family member(s) | Publication date |
|---|---|---|---|
| WO 2005061488 A | | EP 1699782 A1 | 13-09-2006 |
| | | ES 2345776 T3 | 01-10-2010 |
| | | HR 20100455 T1 | 30-09-2010 |
| | | JP 2007517008 T | 28-06-2007 |
| | | KR 20060127899 A | 13-12-2006 |
| | | MX PA06007191 A | 23-08-2006 |
| | | NZ 547547 A | 28-08-2009 |
| | | PT 1699782 E | 04-08-2010 |
| | | SI 1699782 T1 | 30-09-2010 |
| | | US 2007129387 A1 | 07-06-2007 |
| | | US 2010298357 A1 | 25-11-2010 |
| | | UY 28702 A1 | 29-07-2005 |
| | | ZA 200605225 A | 30-05-2007 |
| WO 0047212 A1 | 17-08-2000 | AT 298237 T | 15-07-2005 |
| | | AT 482946 T | 15-10-2010 |
| | | AU 763618 B2 | 31-07-2003 |
| | | BR 0008128 A | 13-02-2002 |
| | | CA 2362715 A1 | 17-08-2000 |
| | | CA 2674803 A1 | 17-08-2000 |
| | | CN 1346271 A | 24-04-2002 |
| | | CN 1597667 A | 23-03-2005 |
| | | CZ 20012889 A3 | 14-11-2001 |
| | | DE 60020941 D1 | 28-07-2005 |
| | | DE 60020941 T2 | 11-05-2006 |
| | | DK 1154774 T3 | 26-09-2005 |
| | | DK 1553097 T3 | 13-12-2010 |
| | | EE 200100409 A | 16-12-2002 |
| | | EP 1154774 A1 | 21-11-2001 |
| | | EP 1553097 A1 | 13-07-2005 |
| | | EP 2050744 A1 | 22-04-2009 |
| | | ES 2242596 T3 | 16-11-2005 |
| | | HK 1041212 A1 | 02-12-2005 |
| | | HK 1076104 A1 | 31-10-2008 |
| | | HU 0104964 A2 | 29-04-2002 |
| | | ID 30552 A | 20-12-2001 |
| | | IL 144745 A | 03-11-2008 |
| | | IS 6022 A | 24-07-2001 |
| | | IS 8708 A | 25-01-2008 |
| | | JP 3893026 B2 | 14-03-2007 |
| | | JP 2002536414 T | 29-10-2002 |
| | | JP 2006273860 A | 12-10-2006 |
| | | KR 20080015482 A | 19-02-2008 |
| | | MX PA01008182 A | 20-08-2003 |
| | | NO 20013882 A | 09-10-2001 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 10 18 2729

This annex lists the patent family members relating to the patent documents cited in the above-mentioned European search report.
The members are as contained in the European Patent Office EDP file on
The European Patent Office is in no way liable for these particulars which are merely given for the purpose of information.

27-12-2010

| Patent document cited in search report | Publication date | Patent family member(s) | Publication date |
|---|---|---|---|
| | | NZ 513204 A | 30-04-2004 |
| WO 0047212 A1 | | NZ 530832 A | 27-05-2005 |
| | | PL 350565 A1 | 16-12-2002 |
| | | PL 205557 B1 | 31-05-2010 |
| | | PT 1154774 E | 31-10-2005 |
| | | SI 1154774 T1 | 31-10-2005 |
| | | SK 11402001 A3 | 07-01-2002 |
| | | TR 200102314 T2 | 21-01-2002 |
| | | TR 200500745 T2 | 23-05-2005 |
| | | UA 73932 C2 | 15-01-2002 |
| | | US 2006004017 A1 | 05-01-2006 |
| | | US 7074800 B1 | 11-07-2006 |
| | | ZA 200106340 A | 01-11-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0047212 A
- WO 05061488 A


### Non-patent literature cited in the description


- **Fan et al.** *Trends Pharmacol. Sci.,* 1995, vol. 16, 57-66
- **Folkman.** *Nature Medicine,* 1995, vol. 1, 27-31
- **Cullinan-Bove et al.** *Endocrinology,* 1993, vol. 133, 829-837
- **Senger et al.** *Cancer and Metastasis Reviews,* 1993, vol. 12, 303-324
- **Jakeman et al.** *Endocrinology,* 1993, vol. 133, 848-859
- **Kolch et al.** *Breast Cancer Research and Treatment,* 1995, vol. 36, 139-155
- **Connolly et al.** *J. Biol. Chem.,* 1989, vol. 264, 20017-20024
- **Kim et al.** *Nature,* 1993, vol. 362, 841-844
- **De Vries et al.** *Science,* 1992, vol. 255, 989-991
- **Terman et al.** *Biochem. Biophys. Res. Comm.,* 1992, vol. 187, 1579-1586
- **Keck, P.J. ; Hauser, S.D. ; Krivi, G. ; Sanzo, K. ; Warren, T. ; Feder, J. ; Connolly, D.T.** *Science,* 1989, vol. 246, 1309-1312
- **Lamoreaux, W.J. ; Fitzgerald, M.E. ; Reiner, A. ; Hasty, K.A. ; Charles, S.T.** *Microvasc. Res.,* 1998, vol. 55, 29-42
- **Pepper, M.S. ; Montesano, R. ; Mandroita, S.J. ; Orci, L. ; Vassalli, J.D.** *Enzyme Protein,* 1996, vol. 49, 138-162
- **Dvorak, H.F. ; Detmar, M. ; Claffey, K.P. ; Nagy, J.A. ; van de Water, L. ; Senger, D.R.** *Int. Arch. Allergy Immunol.,* 1995, vol. 107, 233-235
- **Bates, D.O. ; Heald, R.I. ; Curry, F.E. ; Williams, B.** *J. Physiol. (Lond.),* 2001, vol. 533, 263-272
- **Meyer, M. ; Clauss, M. ; Lepple-Wienhues, A. ; Waltenberger, J. ; Augustin, H.G. ; Ziche, M. ; Lanz, C. ; Büttner, M. ; Rziha, H-J. ; Dehio, C.** *EMBO J.,* 1999, vol. 18, 363-374
- **Zeng, H. ; Sanyal, S. ; Mukhopadhyay, D.** *J. Biol. Chem.,* 2001, vol. 276, 32714-32719
- **Gille, H. ; Kowalski, J. ; Li, B. ; LeCouter, J. ; Moffat, B ; Zioncheck, T.F. ; Pelletier, N. ; Ferrara, N.** *J. Biol. Chem.,* 2001, vol. 276, 3222-3230
- **Wedge et al.** *Cancer Research,* 2005, vol. 65, 4389-4440
- Handbook of Pharmaceutical Excipients. Pharmaceutical Press, 2003
- **Carstensen, J. T.** *Solid pharmaceutics: mechanical properties and rate phenomena,* 1980
- **Sheth et al.** Pharmaceutical dosage forms: Tablets. Pub. Marcel Dekker, 1980, vol. 1
- **Roberts, R. J. ; Rowe, R.C.** The effect of punch velocity on the compaction of a variety of materials. *J. Pharm. Pharmacol.,* 1985, vol. 37, 377-384
- **Rowe, R. C. ; Roberts, R.J.** Advances in Pharmaceutical Sciences. 1995, vol. 7, 34